# EUROPEAN PATENT APPLICATION

(11) **EP 4 574 155 A1**
(43) Date of publication of application: **25.06.2025**
(21) Application number: 23220012.1
(22) Date of filing: 22.12.2023
(51) Int. Cl.: A61K 31/7105, C12N 15/88, C12N 15/113, A61K 38/17, A61K 31/7115, A61P 19/02

(54) **MOLECULES FOR USE IN CELL REGENERATION AND REJUVENATION**

(71) Applicant: Süßmuth, Christine, 28211 Bremen (DE)
(72) Inventor: Süßmuth, Christine, 28211 Bremen (DE)
(74) Representative: Witthoff Jaekel Steinecke Patentanwälte PartG mbB

(57) **Abstract**

Disclosed are molecules encoding HMGA1 or HMGA2 or parts thereof, nanovesicles containing these for use in cell regeneration and rejuvenation as well as pharmaceutical compositions comprising these.

## Description

### TECHNICAL FIELD

The present invention generally relates to molecules encoding HMGA1 or HMGA2 or parts thereof, nanovesicles containing these for use in cell regeneration and rejuvenation.

### BACKGROUND

As to many organs, a reduced ability for tissue repair, rejuvenation, and regeneration are hallmarks of the aging process. Simultaneously, this decreasing ability is accompanied by a reduced quality of life as well as by an enormous burden for the health care systems.

One well-known example is cartilage damage which can have a variety of reasons. One of the most common causes is slow degeneration over time. Nevertheless, *e.g*., injury, excessive weight, excessive activity, or poor alignment of the joint can contribute to the loss of cartilage tissue and disrupt functional properties of this tissue (https://cartilage.org/patient/about-cartilage/what-is-cartilage-damage/). Also, damaged cartilage is a leading sign of autoimmune arthritis as well as of arthrosis. However, treatment strategies allowing to induce a repair of damaged cartilage are still in their infancy. Apparently, the same holds true for repair of damaged or age-dependent loss of other tissues.

Accordingly, there is a requirement for aims for targeted influencing the production of cartilaginous tissue or other highly differentiated tissues and cells such as muscle or bone cells in a regulated manner to either increase or decrease their production.

### SUMMARY OF THE DISCLOSURE

The present invention relates to the embodiments as characterized in the claims, disclosed in the description and illustrated in the Examples and Figures further below. It is aimed herein at the use of genetic mechanisms for repair of cells and tissues as, *e.g.,* bone marrow-derived stem cells, cartilage, muscle, cells of the inner ear, and endometrium. More specifically, preparations containing a coding sequence, in particular mRNA of specific genes are described herein for the delivery to damaged joint and a variety of tissues. The types of these coding sequences, in particular mRNAs and how they can be stabilized and prepared for in vivo delivery is described. Basically, selected coding sequences, such as mRNAs will be prepared according to rules as recently applied for mRNA vaccination with one fundamental difference that it is not the immune system of the treated subject which is targeted. Furthermore, the invention aims at an intracellular delivery followed by expression of the corresponding functional protein which in turn is able to induce the repair of damaged or aged tissue and cells.

As shown for mRNA-based vaccines, stabilized ectopic mRNA becomes transferred to cells of the person to be vaccinated (Bansal et al., 2021; Cari et al., 2023). This procedure aims at the presentation of the corresponding protein by the proband's cells and the induction of an immune response against said protein. In contrast, herein the use of coding sequences, in particular of mRNA is described aimed at the intracellular supplementation of the proteins within its recipient's cells thus triggering cell and tissue repair.

As illustrated in the experiments performed in accordance with the present invention and shown in the Examples, induction of nuclear overexpression of HMGA1 or HMGA2 by transfer of nucleic acids encoding for these or by disruption of the negative effect of miRNAs of the let-7 family on the expression of the endogenous HMGA1 or HMGA2 genes supplemented the intracellular presence of the corresponding proteins within the cells receipting either the HMGA1/HMGA2 encoding sequences or cells in which the let-7-family miRNAs were downregulated, this triggering cell and tissue repair in damaged or aged tissue and cells.

Therefore, provided herein is a nucleic acid encoding HMGA2 or a part thereof for use in cell regeneration. Preferably the nucleic acid encodes a part of HMGA2 containing at least exons 1-3 of the gene, mostly preferred at least exons 1-3 of the cDNA with the SEQ ID No.: 4. Mostly preferred, the nucleic acid is a mRNA.

Furthermore, herein provided is also a nucleic acid encoding HMGA1 or a part thereof for use in cell regeneration. Preferably the nucleic acid encodes a part of HMGA1 containing at least exons 1-3 of the gene, mostly preferred at least exons 1-3 of the cDNA with the SEQ ID No.: 1. Mostly preferred, the nucleic acid is a mRNA.

Preferably, the nucleic acid or mRNA molecule for use in cell regeneration as described herein is provided, wherein the regenerated cells are selected from bone marrow stem cells and cartilage cells.

Mostly preferred, the nucleic acid or mRNA molecule encoding HMGA1 or HMGA2 or a part thereof respectively as defined herein is provided in addition or alternatively for use in cell rejuvenation.

Preferably, the nucleic acid, in particular mRNA molecule as defined herein for use in cell regeneration and/or rejuventation is provided, wherein the regenerated cells are selected from the group consisting of bone marrow stem cells including hematopoietic stem cells and mesenchymal stem cells, cartilage cells, cartilage stem cells, smooth muscle cells, smooth muscle stem cells, hairy cells and stem cells of the inner ear, soft tissue stem cells, and the rejuvenated cells are endometrium cells.

Furthermore, a nucleic acid, preferably a mRNA molecule as defined herein is provided for use in treatment of disease or diseases related to cartilage loss. Preferably, the nucleic acid or mRNA molecule for use in treatment as defined herein is provided, wherein the disease or diseases are selected from the group consisting of arthrose, arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis .

Furthermore, the mRNA molecule for use in treatment as defined herein is provided, wherein at least 10 % uridine of the natural mRNA has been substituted by pseudouridine. Preferably the pseudouridine is N1-methyl-pseudouridine.

In addition, the mRNA molecule for use in treatment as defined herein is provided where uridines of the natural mRNA have been substituted by residues with lower immunogenicity.

Furthermore, the nucleic acid, and preferably the mRNA molecule for use as defined herein is provided, wherein at least one of the let-7 binding sites has been removed or mutated.

In one embodiment the mRNA molecule or modified mRNA molecule for use as defined herein is provided wherein the mRNA is a "self-replicating mRNA" or a "self-amplifying mRNA".

Provided herein is also a nanovesicle (NV) containing at least one nucleic acid, preferably at least one mRNA molecule or modified mRNA molecule as defined herein.

Different types of nanovesicles can be used according to the present invention. Preferably, the nanovesicle containing a nucleic acid, preferably the mRNAs or modified mRNAs as defined herein is provided, wherein the nanovesicle is selected from the group consisting of exosomes, artificial exosomes, lipid-based nanoparticles, liposomes, niosomes, ethosomes, and cell derived nanovesicles.

Furthermore, provided herein is a pharmaceutical composition comprising (as active ingredient) the at least one nucleic acid, preferably the at least one mRNA molecule or modified mRNAs as defined herein, or at least one nanovesicle as defined herein and a pharmaceutically acceptable carrier.

In one embodiment, the present invention further relates generally to a use of a nucleic acid, preferably of a mRNA molecule or modified mRNAs as defined herein or of a nanovesicle as defined herein for regeneration or rejuvenation of cells. Preferably said use is performed in cell culture. Regarding said use, preferably, if performed in cell culture, the cell culture comprises cells selected from the group consisting of bone marrow stem cells including hematopoietic stem cells and mesenchymal stem cells, cartilage, cartilage stem cells, smooth muscle cells, smooth muscle stem cells, hairy cells and stem cells of the inner ear, soft tissue stem cells and endometrial cells.

### BRIEF DESCRIPTION OF THE DRAWINGS

**Figure 1****:** *HMGA1* and HMGA1: Complete cDNA (SEQ ID No.: 1), coding sequence (SEQ ID No.: 2), and amino acid sequence (SEQ ID No.: 3) (first row, second row, and third row, each)
**Figure 2****:** *HMGA1:* Complete cDNA (SEQ ID No.: 1).
**Figure 3****:** *HMGA1:* cDNA, coding sequence (SEQ ID No.: 2).
**Figure 4****:** HMGA1: amino acid sequence (SEQ ID No.: 3).
**Figure 5****:** *HMGA2* and HMGA2: Complete cDNA (SEQ ID No.: 4), coding sequence (SEQ ID No.: 5), and amino acid sequence (SEQ ID No.: 6) (first row, second row, and third row, each)
**Figure 6****:** *HMGA2:* Complete cDNA (SEQ ID No.: 4).
**Figure 7****:** *HMGA2:* cDNA, coding sequence (SEQ ID No.: 5).
**Figure 8****:** HMGA2: amino acid sequence (SEQ ID No.: 6).
**Figure 9****:** Let-7 binding seed sites within the 3'UTR (SEQ ID No.: 7) of *HMGA2* showing several high affinity binding sites for miRNAs of the let-7 family, *i.e*., let-7f-5p, let-7d-5p, let-7b-5p, let-7g-5p, let-7a-5p, let-7i-5p, let-7e-5p, let-7c-5p indicated in blue and underlined. The seed locations (21, 1107, 1256, 1619, 1668, 2521. 2541) apply to the miRNAs as mentioned above.

### DETAILED DESCRIPTION

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (ILTPAC Recommendations)", H.G.W. Leuenberger, B. Nagel, and H. 20 Kolbl, Eds., Helvetica Chimica Acta, CH-4010 Basel, Switzerland, (1995).

The practice of the present disclosure will employ, unless otherwise indicated, conventional methods of chemistry, biochemistry, cell biology, immunology, and recombinant DNA techniques which are explained in the literature in the field (cf, e.g., Molecular Cloning: A Laboratory Manual, 2nd Edition, J. Sambrook et al. eds., Cold Spring Harbor Laboratory Press, Cold Spring Harbor 1989).

For the avoidance of any doubt, it is emphasized that the expressions "in some embodiments", "in a certain embodiments," "in certain instances," "in some instances," "in some aspects," "in a further embodiment," "in one embodiment," "in a further aspect," "in a first aspect," "in a second aspect," etc., and the like are used and meant such that any of the embodiments described therein are to be read with a mind to combine each of the features of those embodiments and that the disclosure has to be treated in the same way as if the combination of the features of those embodiments and aspects would be spelled out in one embodiment. The same is true for any combination of embodiments and features of the appended claims and illustrated in the Examples, which are also intended to be combined with features from corresponding embodiments disclosed in the description, wherein only for the sake of consistency and conciseness the embodiments are characterized by dependencies while in fact each embodiment and combination of features, which could be construed due to the (multiple) dependencies must be seen to be literally disclosed and not considered as a selection among different choices. In this context, the person skilled in the art will appreciate that the embodiments and features disclosed in the Examples are intended to be generalized to any RNA molecule encoding HMGA1, HMGA2 or a part thereof and equivalents having substantially the same properties.

The term "about" means approximately or nearly, and in the context of a numerical value or range set forth herein in some embodiments means± 20%, ± 10%, ± 5%, or ± 3% of the numerical value or range recited or claimed. As used herein, the term "about", as used herein, preferably refers to a value that is ± 10%, more preferably ± 5%, mostly preferred ± 3% of a recited value.

The terms "a" and "an" and "the" and similar reference used in the context of describing the disclosure (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (*e.g*., "such as"), provided herein is intended merely to better illustrate the disclosure and does not pose a limitation on the scope of the claims. No language in the specification should be construed as indicating any non-claimed element essential to the practice of the disclosure.

Unless expressly specified otherwise, the term "comprising" is used in the context of the present document to indicate that further members may optionally be present in addition to the members of the list introduced by "comprising". It is, however, contemplated as a specific embodiment of the present disclosure that the term "comprising" encompasses the possibility of no further members being present, *i.e.,* for the purpose of this embodiment "comprising" is to be understood as having the meaning of "consisting of" or "consisting essentially of'.

In connection with the present invention, the term "and/or" is understood to mean that all members of a group which are connected by the term "and/or" are disclosed cumulatively in any combination, both alternatively to each other and in each case to each other. This means for the expression "A, B and/or C" that the following disclosure content is to be understood thereunder: a) A or B or C; or b) (A and B); or c) (A and C); or d) (B and C); or e) (A and B and C).

***Agent:*** As used herein, the term "agent", may refer to a physical entity or phenomenon. In some embodiments, an agent may be characterized by a particular feature and/or effect. In some embodiments, an agent may be a compound, molecule, or entity of any chemical class including, for example, a small molecule, polypeptide, nucleic acid, saccharide, lipid, metal, or a combination or complex thereof. In some embodiments, the term "agent" may refer to a compound, molecule, or entity that comprises a polymer. In some embodiments, the term may refer to a compound or entity that comprises one or more polymeric moieties. In some embodiments, the term "agent" may refer to a compound, molecule, or entity that is substantially free of a particular polymer or polymeric moiety. In some embodiments, the term may refer to a compound, molecule, or entity that lacks or is substantially free of any polymer or polymeric moiety.

***Amino acid:*** in its broadest sense, as used herein, the term "amino acid" refers to a compound and/or substance that can be, is, or has been incorporated into a polypeptide chain, *e.g.,* through formation of one or more peptide bonds. In some embodiments, an amino acid has the general structure H2N-C(H)(R)-COOH. In some embodiments, an amino acid is a naturally-occurring amino acid. In some embodiments, an amino acid is a non-natural amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. In some embodiments, an amino acid, including a carboxy and/or amino-terminal amino acid in a polypeptide, can contain a structural modification as compared with the general structure above. For example, in some embodiments, an amino acid may be modified by methylation, amidation, acetylation, pegylation, glycosylation, phosphorylation, and/or substitution *(e.g.,* of the amino group, the carboxylic acid group, one or more protons, and/or the hydroxyl group) as compared with the general structure. In some embodiments, such modification may, for example, alter the circulating half-life of a polypeptide containing the modified amino acid as compared with one containing an otherwise identical unmodified amino acid. In some embodiments, such modification does not significantly alter a relevant activity of a polypeptide containing the modified amino acid, as compared with one containing an otherwise identical unmodified amino acid. As will be clear from context, in some embodiments, the term "amino acid" may be used to refer to a free amino acid; in some embodiments it may be used to refer to an amino acid residue of a polypeptide.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, *e.g*., by chemical manipulation of the reference substance. In some embodiments, an analog is a substance that can be generated through performance of a synthetic process substantially similar to (*e.g*., sharing a plurality of steps with) one that generates the reference substance. In some embodiments, an analog is or can be generated through performance of a synthetic process different from that used to generate the reference substance.

***Associated:*** Two events or entities are "associated" with one another, as that term is used herein, if the presence, level, degree, type and/or form of one is correlated with that of the other. For example, a particular entity *(e.g.,* polypeptide, genetic signature, metabolite, microbe, etc) is considered to be associated with a particular disease, disorder, or condition, if its presence, level and/or form correlates with incidence of, susceptibility to, severity of, stage of, etc the disease, disorder, or condition *(e.g.,* across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Combination therapy:*** As used herein, the term "combination therapy" refers to those situations in which a subject is simultaneously exposed to two or more therapeutic regimens *(e.g.,* two or more therapeutic agents). In some embodiments, the two or more regimens may be administered simultaneously; in some embodiments, such regimens may be administered sequentially *(e.g.,* all "doses" of a first regimen are administered prior to administration of any doses of a second regimen); in some embodiments, such agents are administered in overlapping dosing regimens. In some embodiments, "administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although in some embodiments, two or more agents, or active moieties thereof, may be administered together in a combination composition, or even in a combination compound *(e.g.,* as part of a single chemical complex or covalent entity).

***Complementary:*** As used herein, the term "complementary" is used in reference to oligonucleotide hybridization related by base-pairing rules. For example, the sequence "C-A-G-T" is complementary to the sequence "G-T-C-A." Complementarity can be partial or total. Thus, any degree of partial complementarity is intended to be included within the scope of the term "complementary" provided that the partial complementarity permits oligonucleotide hybridization. Partial complementarity is where one or more nucleic acid bases is not matched according to the base pairing rules. Total or complete complementarity between nucleic acids is
where each and every nucleic acid base is matched with another base under the base pairing rules.

***Comparable:*** As used herein, the term "comparable" refers to two or more agents, entities, situations, sets of conditions, etc., that may not be identical to one another but that are sufficiently similar to permit comparison there between so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Corresponding to:*** As used herein, the term "corresponding to" refers to a relationship between two or more entities. For example, the term "corresponding to" may be used to designate the position/identity of a structural element in a compound or composition relative to another compound or composition *(e.g.,* to an appropriate reference compound or composition). For example, in some embodiments, a monomeric residue in a polymer *(e.g.,* an amino acid residue in a polypeptide or a nucleic acid residue in a polynucleotide) may be identified as "corresponding to" a residue in an appropriate reference polymer. For example, those of ordinary skill will appreciate that, for purposes of simplicity, residues in a polypeptide are often designated using a canonical numbering system based on a reference related polypeptide, so that an amino acid *"corresponding to"* a residue at position 190, for example, need not actually be the 190^{th} amino acid in a particular amino acid chain but rather corresponds to the residue found at 190 in the reference polypeptide; those of ordinary skill in the art (person skilled in the art) readily appreciate how to identify *"corresponding"* amino acids. For example, those skilled in the art will be aware of various sequence alignment strategies, including software programs such as, for example, BLAST, CS-BLAST, CUSASW++, DIAMOND, FASTA, GGSEARCH/GLSEARCH, Genoogle, HMMER, HHpred/HHsearch, IDF, Infernal, KLAST, USEARCH, parasail, PSIBLAST, PSI-Search, ScalaBLAST, Sequilab, SAM, SSEARCH, SW APHI, SW APHI-LS, SWIMM, or SWIPE that can be utilized, for example, to identify "corresponding" residues in polypeptides and/or nucleic acids in accordance with the present disclosure. Those of skill in the art will also appreciate that, in some instances, the term "corresponding to" may be used to describe an event or entity that shares a relevant similarity with another event or entity *(e.g.,* an appropriate reference event or entity). To give but one example, a gene or protein in one organism may be described as "corresponding to" a gene or protein from another organism in order to indicate, in some embodiments, that it plays an analogous role or performs an analogous function and/or that it shows a particular degree of sequence identity or homology, or shares a particular characteristic sequence element.

***Determination of similiarity and*/*or identity of molecules:*** "Similarity" between two peptides is determined by comparing the amino acid sequence of one peptide to the sequence of a second peptide. An amino acid of one peptide is similar to the corresponding amino acid of a second peptide if it is identical or a conservative amino acid substitution. Conservative substitutions include those described in Dayhoff, M.O., ed., The Atlas of Protein Sequence and Structure 5, National Biomedical Research Foundation, Washington, D.C. (1978), and in Argos, EMBO J. 8 (1989), 779-785. For example, amino acids belonging to one of the following groups represent conservative changes or substitutions: -Ala, Pro, Gly, Gln, Asn, Ser, Thr; -Cys, Ser, Tyr, Thr; -Val, Ile, Leu, Met, Ala, Phe; -Lys, Arg, His; -Phe, Tyr, Trp, His; and -Asp, Glu.

The determination of percent identity or similarity between two sequences is preferably accomplished using the mathematical algorithm of Karlin and Altschul (1993) Proc. Natl. Acad. Sci USA 90: 5873-5877. Such an algorithm is incorporated into the BLASTn and BLASTp programs of Altschul et al. (1990) J. Mol. Biol. 215: 403-410 available at NCBI (http://www.ncbi.nlm.nih.gov/blast/Blast.cge).

The determination of percent identity or similarity is performed with the standard parameters of the BLASTn and BLASTp programs, as recommended on the NCBI webpage and in the "BLAST Program Selection Guide" in respect of sequences of a specific length and composition.

BLAST polynucleotide searches are performed with the BLASTn program.
For the general parameters, the "Max Target Sequences" box may be set to 100, the "Short queries" box may be ticked, the "Expect threshold" box may be set to 1000 and the "Word Size" box may be set to 7 as recommended for short sequences (less than 20 bases) on the NCBI webpage. For longer sequences the "Expect threshold" box may be set to 10 and the "Word Size" box may be set to 11. For the scoring parameters the "Match/mismatch Scores" may be set to 1,-2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "DUST Filter Settings" may be ticked and the "Mask lower case letters" box may not be ticked. In general the "Search for short nearly exact matches" may be used in this respect, which provides most of the above indicated settings. Further information in this respect may be found in the "BLAST Program Selection Guide" published on the NCBI webpage.

BLAST protein searches are performed with the BLASTp program. For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension: 1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

Modifications of both programs, e.g., in respect of the length of the searched sequences, are performed according to the recommendations in the "BLAST Program Selection Guide" published in a HTML and a PDF version on the NCBI webpage.

***Encode:*** As used herein, the term "encode(s)" or "encoding" refers to sequence information of a first molecule that guides production of a second molecule having a defined sequence of nucleotides *(e.g.,* mRNA) or a defined sequence of amino acids. For example, a DNA molecule can encode an RNA molecule *(e.g.,* by a transcription process that includes a DNA-dependent RNA polymerase enzyme). An RNA molecule can encode a polypeptide *(e.g.,* by a translation process). Thus, a gene, a cDNA, or a single-stranded RNA *(e.g.,* an mRNA) encodes a polypeptide if transcription and translation of mRNA corresponding to that gene produces the polypeptide in a cell or other biological system. In some embodiments, a coding region of a single-stranded RNA encoding a target polypeptide agent refers to a coding strand, the nucleotide sequence of which is identical to the mRNA sequence of such a target polypeptide agent. In some embodiments, a coding region of a single-stranded RNA encoding a target polypeptide agent refers to a non-coding strand of such a target polypeptide agent, which may be used as a template for transcription of a gene or cDNA.

***Exosomes:*** In this respect vesicles or exosomes may be used as well. "Exosomes" are vesicles of endosomal origin of about 30-100 nm that are secreted in the extracellular milieu following fusion of late endosomal multivesicular bodies with the plasma membrane (Garin et al., J Cell Biol 152 (2001), 165-180). Cells from various tissue types have been shown to secrete exosomes, such as dendritic cells, B lymphocytes, tumor cells and mast cells, for instance. Exosomes from different origin exhibit discrete sets of proteins and lipid moieties (J Thery et al., Cell Biol 147 (1999), 599-610; Thery et al., J Immunol 166 (2001), 7309-7318). They notably contain proteins involved in antigen presentation and immunomodulation suggesting that exosomes play a role in cell-cell communications (Simons and Raposo, Curr. Opin. Cell Biol. 21 (2009), 575-581; Thery et al., Nat. Rev. Immunol. 9 (2009), 581-593) leading to the modulation of immune responses. Methods of producing, purifying or using exosomes for therapeutic purposes or as research tools have been described for instance in WO99/03499, WO00/44389 and WO97/05900, the disclosure content of which is incorporated herein by reference. Furthermore, methods for producing artificial exosomes are known in the art as well. Such artificial endosomes can be derived from coated liposomes as described in De La Peña et al., J Immunol Methods. 344 (2009), 121-132.

Considering their immunogenic and therapeutic properties, it would be particularly useful to be able to modify the content of exosomes in order to alter their properties. In this respect, recombinant exosomes have been described in the art, which derive from cells transfected with plasmids encoding recombinant proteins. Such recombinant exosomes contain the plasmid-encoded recombinant protein (WO00/28001).

***Expression:*** As used herein, the term "expression" of a nucleic acid sequence refers to the generation of any gene product from the nucleic acid sequence. In some embodiments, a gene product can be a transcript. In some embodiments, a gene product can be a polypeptide. In some embodiments, expression of a nucleic acid sequence involves one or more of the following: (1) production of an RNA template from a DNA sequence *(e.g.,* by transcription); (2) processing of an RNA transcript *(e.g.,* by splicing, editing, etc); (3) translation of an RNA into a polypeptide or protein; and/or ( 4) post-translational modification of a polypeptide or protein. In a preferred embodiment the term expression may also refer to only steps (3) and (4) mentioned above, in particular regarding expression from a mRNA molecule or part thereof as defined herein.

***Improved, increased* or *reduced:*** As used herein, these terms, or grammatically comparable comparative terms, indicate values that are relative to a comparable reference measurement. For example, in some embodiments, an assessed value achieved with an agent of interest may be "improved" relative to that obtained with a comparable reference agent. Alternatively or additionally, in some embodiments, an assessed value achieved in a subject or system of interest may be "improved" relative to that obtained in the same subject or system under different conditions *(e.g.,* prior to or after an event such as administration of an agent of interest), or in a different, comparable subject *(e.g.,* in a comparable subject or system that differs from the subject or system of interest in presence of one or more indicators of a particular disease, disorder or condition of interest, or in prior exposure to a condition or agent, *etc.).* In some embodiments, comparative terms refer to statistically relevant differences *(e.g.,* that are of a prevalence and/or magnitude sufficient to achieve statistical relevance). Those skilled in the art will be aware, or will readily be able to determine, in a given context, a degree and/or prevalence of difference that is required or sufficient to achieve such statistical significance.

***In vitro:*** The term *"in vitro"* as used herein refers to events that occur in an artificial environment, *e.g.,* in a test tube or reaction vessel *(e.g.,* a bioreactor), in cell culture, *etc.,* rather than within a multi-cellular organism.

***Pharmaceutical composition:*** As used herein, the term "pharmaceutical composition" refers to an active agent, formulated together with one or more pharmaceutically acceptable carriers. In some embodiments, active agent is present in unit dose amount appropriate for administration in a therapeutic regimen that shows a statistically significant probability of achieving a predetermined therapeutic effect when administered to a relevant population. In some embodiments, pharmaceutical compositions may be specially formulated for parenteral administration, for example, by subcutaneous, intramuscular, intravenous or epidural injection as, for example, a sterile solution or suspension, or sustained-release formulation.

***Prevent* or *prevention:*** as used herein when used in connection with the occurrence of a disease, disorder, and/or condition, refers to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Pure or Purified:*** As used herein, an agent or entity is "pure" or "purified" if it is substantially free of other components. For example, a preparation that contains more than about 90% of a particular agent or entity is typically considered to be a pure preparation. In some embodiments, an agent or entity is at least 91 %, at least 92%, at least 93%, at least 94%, at least 95%, at least 96%, at least 97%, at least 98%, or at least 99% pure in a preparation.

***Reference:*** As used herein describes a standard or control relative to which a comparison is performed. For example, in some embodiments, an agent, animal, individual, population, sample, sequence or value of interest is compared with a reference or control agent, animal, individual, population, sample, sequence or value. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Ribonucleotide:*** As used herein, the term "ribonucleotide" encompasses unmodified ribonucleotides and modified ribonucleotides. For example, unmodified ribonucleotides include the purine bases adenine (A) and guanine (G), and the pyrimidine bases cytosine (C) and uracil (U). Modified ribonucleotides may include one or more modifications including, but not limited to, for example, (a) end modifications, *e.g.,* 5' end modifications (*e.g*., phosphorylation, dephosphorylation, conjugation, inverted linkages, *etc.),* 3' end modifications *(e.g.,* conjugation, inverted linkages, *etc.),* (b) base modifications, *e.g.* , replacement with modified bases, stabilizing bases, destabilizing bases, or bases that base pair with an expanded repertoire of partners, or conjugated bases, (c) sugar modifications *(e.g.,* at the 2' position or 4' position) or replacement of the sugar, and ( d) intemucleoside linkage modifications, including modification or replacement of the phosphodiester linkages. The term "ribonucleotide" also encompasses ribonucleotide triphosphates including modified and non-modified ribonucleotide triphosphates.

***RNA polynucleotides:*** The term "polynucleotide" or "nucleic acid", as used herein, refers to DNA and RNA such as genomic DNA, cDNA, mRNA, recombinantly produced and chemically synthesized molecules. A nucleic acid may be single-stranded or double-stranded. RNA includes *in vitro* transcribed RNA (IVT RNA) or synthetic RNA According to the invention, a polynucleotide is preferably isolated.

In some embodiments, a nucleic acid as described and/or utilized herein may be or comprise recombinant and/or isolated molecules. Those skilled in the art, reading the present disclosure, will understand that the term "RNA" typically refers to a nucleic acid molecule which includes ribonucleotide residues. In some embodiments, an RNA contains all or a majority of ribonucleotide residues. As used herein, "ribonucleotide" refers to a nucleotide with a hydroxyl group at the 2'-position of a β-D-ribofuranosyl group. In some embodiments, an RNA may be partly or fully double stranded RNA; in some embodiments, an RNA may comprise two or more distinct nucleic acid strands (*e.g*., separate molecules) that are partly or fully hybridized with one another. In many embodiments, an RNA is a single strand, which may in some embodiments, self-hybridize or otherwise fold into secondary and/or tertiary structures. In some embodiments, an RNA as described and/or utilized herein does not self-hybridize, at least with respect to certain sequences as described herein. In some embodiments, an RNA may be an isolated RNA such as partially purified RNA, essentially pure RNA, synthetic RNA, recombinantly produced RNA, and/or a modified RNA (where the term "modified" is understood to indicate that one or more residues or other structural elements of the RNA differs from naturally occurring RNA; for example, in some embodiments, a modified RNA differs by the addition, deletion, substitution and/or alteration of one or more nucleotides and/or by one or more moieties or characteristics of a nucleotide- *e.g.,* of a nucleoside or of a backbone structure or linkage). In some embodiments, a modification may be or comprise addition of non-nucleotide material to internal RNA nucleotides or to the end(s) of RNA It is also contemplated herein that nucleotides in RNA (*e.g.,* in a modified RNA) may be non-standard nucleotides, such as chemically synthesized nucleotides or deoxynucleotides. For the present disclosure, these altered RNAs are considered analogs of naturally-occurring RNA
As appreciated by a skilled artisan in the art, the RNA polynucleotides disclosed herein can comprise or consist of naturally occurring ribonucleotides and/or modified ribonucleotides. Therefore, a skilled artisan in the art will understand references to A, U, G, or C throughout the specification described herein can refer to a naturally occurring ribonucleotide and/or a modified ribonucleotide described herein. For example, in some embodiments, a U is uridine. N1-methylpseudouridine (m1Ψ) nucleoside modification increases biological stability of RNAs comprising it and thereby the durability of the encoded protein can be monitored longer compared to unmodified RNAs, as reported in Karik6 et al. (2008) Molecular Therapy: the Journal of the American Society of Gene Therapy. Therefore, in some embodiments, a U is modified uridine *(e.g.,* pseudouridine, 1-methyl pseudouridine).

In preferred embodiments of the present disclosure, an RNA is or comprises messenger RNA (mRNA) that relates to an RNA transcript which encodes a polypeptide. In some embodiments, an RNA disclosed herein comprises: a 5' cap comprising a 5' untranslated region comprising a cap proximal sequence (5'-UTR), a sequence encoding a payload *(e.g.,* a polypeptide); a 3' untranslated region (3'-UTR); and/or a polyadenylate (Poly A) sequence.

In some embodiments, an RNA disclosed herein comprises the following components in 5' to 3' orientation: a 5' cap comprising a 5' untranslated region comprising a cap proximal sequence (5'-UTR), a sequence encoding a payload *(e.g.,* a polypeptide); a 3' untranslated region (3'-UTR); and a Poly A sequence.

In some embodiments, an RNA is produced by *in vitro* transcription or chemical synthesis. In some embodiments, an mRNA is produced by *in vitro* transcription using a DNA template where DNA refers to a nucleic acid that contains deoxyribonucleotides. In some embodiments, an RNA disclosed herein is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

In some embodiments, an RNA, respectively mRNA for use as disclosed herein, is "replicon RNA" or simply a "replicon", in particular "self-replicating RNA" or "self-amplifying RNA", or "self-replicating mRNA" or "self-amplifying mRNA" for uses as defined herein respectively. In some embodiments, a replicon or self-replicating RNA/mRNA is derived from or comprises elements derived from a ssRNA virus, in particular a positive-stranded ssRNA virus such as an alphavirus. Alphaviruses are typical representatives of positive-stranded RNA viruses. Alphaviruses replicate in the cytoplasm of infected cells (for review of the alphaviral life cycle see Jose et al., Future Microbial., 2009, vol. 4, pp. 837-856). The total genome length of many alphaviruses typically ranges between 11,000 and 12,000 nucleotides, and the genomic RNA typically has a 5' -cap, and a 3' poly(A) tail. The genome of alphaviruses encodes non-structural proteins (involved in transcription, modification, and replication of viral RNA and in protein modification) and structural proteins (forming the virus particle). There are typically two open reading frames (ORFs) in the genome. The four non-structural proteins (nsPl-nsP4) are typically encoded together by a first ORF beginning near the 5' terminus of the genome, while alphavirus structural proteins are encoded together by a second ORF which is found downstream of the first ORF and extends near the 3' terminus of the genome. Typically, the first ORF is larger than the second ORF, the ratio being roughly 2:1. In cells infected by an alphavirus, only the nucleic acid sequence encoding non-structural proteins is translated from the genomic RNA, while the genetic information encoding structural proteins is translatable from a subgenomic transcript, which is an RNA polynucleotide that resembles eukaryotic messenger RNA (mRNA; Gould et al., 2010, Antiviral Res., vol. 87 pp. 111-124).

Following infection, i.e., at early stages of the viral life cycle, the (+) stranded genomic RNA directly acts like a messenger RNA for the translation of the open reading frame encoding the non-structural poly-protein (nsP1234). Alphavirus-derived vectors have been proposed for delivery of foreign genetic information into target cells or target organisms. In simple approaches, the open reading frame encoding alphaviral structural proteins is replaced by an open reading frame encoding a protein of interest. Alphavirus-based trans-replication systems rely on alphavirus nucleotide sequence elements on two separate nucleic acid molecules: one nucleic acid molecule encodes a viral replicase, and the other nucleic acid molecule is capable of being replicated by said replicase in trans (hence the designation trans-replication system). Trans-replication requires the presence of both these nucleic acid molecules in a given host cell. The nucleic acid molecule capable of being replicated by the replicase in trans must comprise certain alphaviral sequence elements to allow recognition and RNA synthesis by the alphaviral replicase. In some embodiments, the nanovesicles or pharmaceutical compositions as defined herein comprise such "self-replicating mRNA" or "self-amplifying mRNA", as well as the uses described herein refer to the use of such "self-replicating mRNA" or "self-amplifying mRNA".

In some embodiments, an RNA described herein may have modified nucleosides. In some embodiments, an RNA comprises a modified nucleoside in place of at least one *(e.g.,* every) uridine.

The term "uracil," as used herein, describes one of the nucleobases that can occur in the nucleic acid of RNA. The structure of uracil is:

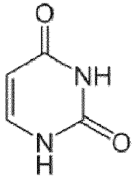

The term "uridine," as used herein, describes one of the nucleosides that can occur in RNA The structure of uridine is:

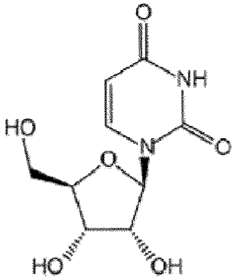

UTP (uridine 5'-triphosphate) has the following structure:

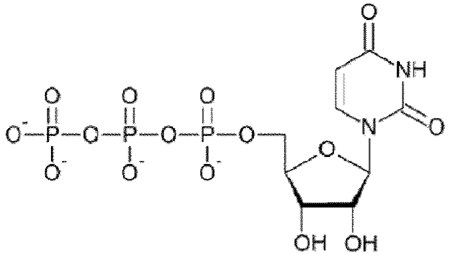

Pseudo-UTP (pseudouridine-5'-triphosphate) has the following structure:

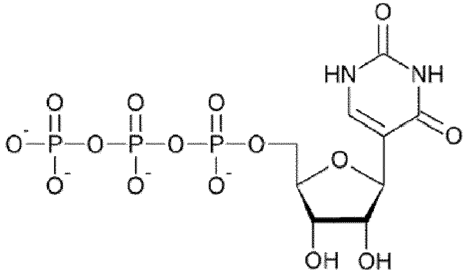

Pseudouridine" is one example of a modified nucleoside that is an isomer of uridine, where the uracil is attached to the pentose ring via a carbon-carbon bond instead of a nitrogen-carbon glycosidic bond.

Another exemplary modified nucleoside is Nl-methylpseudouridine (m1Ψ), which has the structure:

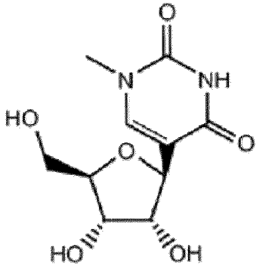

Nl-methylpseudouridine-5'-triphosphate (m1ΨTP) has the following structure:

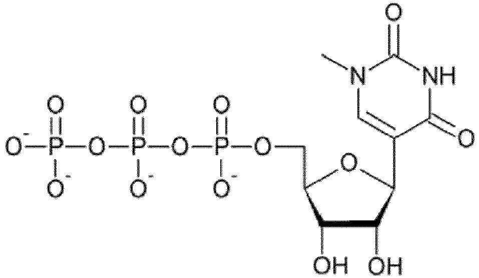

Another exemplary modified nucleoside is 5-methyluridine (m5U), which has the structure:

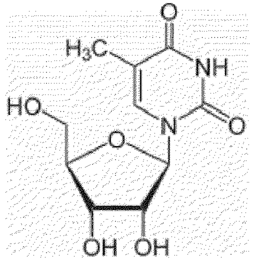

In some embodiments, one or more uridine in an RNA described herein is replaced by a modified nucleoside. In some embodiments, a modified nucleoside is a modified uridine. In some embodiments, an RNA comprises a modified nucleoside in place of at least one uridine. In some embodiments, an RNA comprises a modified nucleoside in place of each uridine.

In some embodiments, a modified nucleoside is independently selected from pseudouridine (Ψ), N1-methylpseudouridine (m1Ψ), and 5-methyluridine (m5U). In some embodiments, a modified nucleoside comprises pseudouridine ('P). In some embodiments, a modified nucleoside comprises N1-methyl-pseudouridine (m1Ψ). In some embodiments, a modified nucleoside comprises 5-methyluridine (m5U). In some embodiments, an RNA may comprise more than one type of modified nucleoside, and a modified nucleosides are independently selected from pseudouridine (Ψ), N1-methylpseudouridine (m1Ψ), and 5- methyluridine (m5U). In some embodiments, a modified nucleosides comprise pseudouridine (Ψ) and N1-methylpseudouridine (m1Ψ). In some embodiments, a modified nucleosides comprise pseudouridine (Ψ) and 5-methyluridine (m5U). In some embodiments, a modified nucleosides comprise N1-methylpseudouridine (m1Ψ) and 5-methyluridine (m5U). In some embodiments, a modified nucleosides comprise pseudouridine (Ψ), N1-methylpseudouridine (m1Ψ), and 5-methyluridine (m5U).

In some embodiments, a modified nucleoside replacing one or more, *e.g.,* all, uridine in the RNA may be any one or more of 3-methyl-uridine (m³U), 5-methoxy-uridine (mo^{S}U), 5-azauridine, 6-aza-uridine, 2-thio-5-aza-uridine, 2-thio-uridine (s²U), 4-thio-uridine (s⁴U), 4-thiopseudouridine, 2-thio-pseudouridine, 5-hydroxy-uridine (ho^{S}U), 5-aminoallyl-uridine, 5-halo-uridine *(e.g.,* 5-iodo-uridine or 5-bromo-uridine), uridine 5-oxyacetic acid (cmo⁵U), uridine 5-oxyacetic acid methyl ester (mcmo⁵U), 5-carboxymethyl-uridine (cm⁵U), 1-carboxymethylpseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5- methoxycarbonylmethyl-2-thio-uridine (mcm⁵s²U), 5-aminomethyl-2-thio-uridine (nm⁵s²U), 5-methylaminomethyl-uridine (mnm⁵U), 1-ethyl-pseudouridine, 5-methylaminomethyl-2-thiouridine (mnm⁵s²U), 5-methylaminomethyl-2-seleno-uridine (mnm⁵se²U), 5-carbamoylmethyluridine (ncm⁵U), 5-carboxymethylaminomethyl-uridine (cmnm⁵U), 5- carboxymethylaminomethyl-2-thio-uridine (cmnm⁵s²U), 5-propynyl-uridine, 1-propynylpseudouridine, 5-taurinomethyl-uridine (τm⁵U), 1-taurinomethyl-pseudouridine, 5-taurinomethyl-2-thio-uridine(τm⁵s²U), l-taurinomethyl-4-thio-pseudouridine, 5-methyl-2-thiouridine (m⁵s²U), l-methyl-4-thio-pseudouridine (m¹s⁴ψ), 4-thio-1-methyl-pseudouridine, 3-methyl-pseudouridine (m³ψ), 2-thio-1-methyl-pseudouridine, l-methyl-1-deaza-pseudouridine, 2-thio-l-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), l-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s²U), α-thio-uridine, 2'-0-methyl-uridine (Um), 5,2'-0-dimethyl-uridine (m⁵Um), 2'-0-methyl-pseudouridine (ψm), 2-thio-2'-0-methyl-uridine (s²Um), 5-methoxycarbonylmethyl-2'-0-methyl-uridine (mcm⁵Um), 5-carbamoylmethyl-2'-0-methyl-uridine (ncm⁵Um), 5-carboxymethylaminomethyl-2'-0-methyl-uridine (cmnm⁵Um), 3,2'-0-dimethyl-uridine (m³Um), 5-(isopentenylaminomethyl)-2'-0-methyl-uridine (inm⁵Um), 1-thio-uridine, deoxythymidine, 2'F-ara-uridine, 2'-F-uridine, 2'-0H-ara-uridine, 5-(2-carbomethoxyvinyl) uridine, 5-[3-(l-E-propenylamino)uridine, or any other modified uridine known in the art.

In some embodiments of the present disclosure, the RNA (such as mRNA) contains one or more modifications, e.g., in order to increase its stability and/or increase translation efficiency and/or decrease immunogenicity and/or decrease cytotoxicity. For example, in order to increase expression of the RNA (such as mRNA), it may be modified within the coding region, *i.e.,* the sequence encoding the expressed peptide or protein, preferably without altering the sequence of the expressed peptide or protein. Such modifications are described, for example, in WO 2007/036366 and PCT/EP2019/056502, and include the following: a 5'-cap structure; an extension or truncation of the naturally occurring poly(A) tail; an alteration of the 5'- and/or 3'-untranslated regions (UTR) such as introduction of a UTR which is not related to the coding region of said RNA; the replacement of one or more naturally occurring nucleotides with synthetic nucleotides (examples of modified ribonucleotides are given below and include, without limitation, 5-methylcytidine, pseudouridine (Ψ), Nl-methyl-pseudouridine (m1ψ) or 5-methyl-uridine (m5U)); and codon optimization *(e.g.,* to alter, preferably increase, the GC content of the RNA). The term "modification" in the context of modified RNA (such as modified mRNA) according to the present disclosure preferably relates to any modification of an RNA (such as an mRNA of the present invention) which is not naturally present in said RNA (such as mRNA).

According to the present invention, the term "transcription" comprises *"in vitro* transcription", wherein the term *"in vitro* transcription" relates to a process wherein RNA, in particular mRNA, is *in vitro* synthesized in a cell-free system, preferably using appropriate cell extracts. Preferably, cloning vectors are applied for the generation of transcripts. These cloning vectors are generally designated as transcription vectors and are according to the present invention encompassed by the term "vector". According to the present invention, the RNA used in the present invention preferably is *in vitro* transcribed RNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription according to the invention is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

With respect to RNA, the term "expression" or "translation" relates to the process in the ribosomes of a cell by which a strand of mRNA directs the assembly of a sequence of amino acids to make a peptide or protein.

In some embodiments, after administration of an RNA described herein, e.g., formulated as RNA lipid particles, respectively Nanovesicles comprising said mRNA encoding HMGA1 or HMGA2 or a portion thereof as defined herein, at least a portion of the RNA is delivered to a target cell. In some embodiments, at least a portion of the RNA is delivered to the cytosol of the target cell. Preferably, the RNA is translated by the target cell to produce the peptide or protein it encodes.

RNA particles such as RNA lipid particles (Nanovesicles) described herein may be used for delivering mRNA to such target cell. Accordingly, the present disclosure also relates to a method for delivering RNA to a target cell in a subject comprising the administration of the RNA particles described herein to the subject. In some embodiments, the RNA is delivered to the cytosol of the target cell. Preferably, the mRNA encoding HMGA1 or HMGA2 or a portion thereof as defined herein is translated by the target cell to produce the peptide or protein encoded by the RNA. "Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e*., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

In one preferred embodiment, nucleic acid compositions described herein, *e.g.,* compositions comprising a lipid nanoparticle encapsulated mRNA (Nanovesicles comprising said mRNA) are characterized by *(e.g.,* when administered to a subject) sustained expression of an encoded polypeptide. For example, in some embodiments, such compositions are characterized in that, when administered to a subject (preferably an animal or human), they achieve detectable polypeptide expression in a biological sample *(e.g.,* serum) from such subject and, in some embodiments, such expression persists for a period of time that is at least at least 36 hours or longer, including, *e.g.,* at least 48 hours, at least 60 hours, at least 72 hours, at least 96 hours, at least 120 hours, at least 148 hours, or longer.

As used herein "endogenous" refers to any material from or produced inside an organism, cell, tissue or system. As used herein, the term "exogenous" refers to any material introduced from or produced outside an organism, cell, tissue or system. The term "expression" as used herein is defined as the transcription and/or translation of a particular nucleotide sequence, preferably of a mRNA molecule encoding HMGA1 or HMGA2 or a part thereof as defined herein.

**5' cap:** A structural feature of mRNAs is cap structure at five-prime end (5'). Natural eukaryotic mRNA comprises a 7-methylguanosine cap linked to the mRNA via a 5' to 5' -triphosphate bridge resulting in capo structure (m7GpppN). In most eukaryotic mRNA and some viral mRNA, further modifications can occur at the 2'-hydroxy-group (2'-0H) *(e.g.,* the 2'-hydroxyl group may be methylated to form 2'-0-Me) of the first and subsequent nucleotides producing "capl" and "cap2" five-prime ends, respectively. Diamond, et al., (2014) Cytokine & growth Factor Reviews, 25:543-550 reported that capO-mRNA cannot be translated as efficiently as capl-mRNA in which the role of 2'-0-Me in the penultimate position at the mRNA 5' end is determinant. Lack of the 2'-0-met has been shown to trigger innate immunity and activate IFN response. Daffis, et al. (2010) Nature, 468:452-456; and Zust et al. (2011) Nature Immunology, 12: 137-143.

RNA capping is well researched and is described, *e.g.,* in Decroly E et al. (2012) Nature Reviews 10: 51-65; and in Ramanathan A et al., (2016) Nucleic Acids Res; 44(16): 7511- 7526, the entire contents of each of which is hereby incorporated by reference. In some embodiments, to imitate the 5' cap structure of natural mRNA, in vitro-transcribed mRNA (IVT mRNA) can be capped either post-transcriptionally using recombinant *Vaccinia* virus-derived enzymes (see., *e.g.,* Kyrieleis, et al. (1993) Structure 22:452-465; and Corbett, et al. (2020) The New England Journal of Medicine 383:1544-1555) or co-transcriptionally by adding caps immediately into the *in vitro* transcription reaction (see, *e.g.,* Jemielity, et al. (2003) RNA 9: 1108-1122; and Kocmik, et al. (2018) Cell Cycle 17: 1624-1636). In some embodiments, enzymatic capping can yield capl-mRNA, but can be time-consuming since it requires an extra purification step, additionally demands a heating step to improve the accessibility of structured 5' ends, thereby increasing further the risk of RNA degradation. Among other things, capping with this method is highly reproducible and less expensive than enzymatic capping. mRNA generated in the presence of these caps are more resistance to the human decapping enzymes (Kowalska et al. (2008) RNA 14: 1119-1131) and/or interferon-induced proteins with tetratricopeptide repeats (IFITs) which inhibits capO-dependent translation (Diamond et al. (2014) Cytokine & Growth Factor Reviews 25:543-550; and Miedziak, et al. (2019) RNA 26:58-68). However, using this approach, GTP is typically competing with caps during transcription that can lead to poor capping efficiency resulting in weak translational capacity. Certain capl structures can be incorporated into IVT mRNA in the right orientation for producing cap 1-mRNA with high capping efficiency in a rapid co-transcriptional reaction. Henderson et al., (2021) Current Protocols l:e39. For example, a trinucleotide capl structure requires AG initiator, avoiding the slippage of RNA polymerases on template DNA strand as opposed to those contain a G triplet as a transcriptional start site. Imburgia, et al. (2000) Biochemistry 20 39: 10419-10430.

In some embodiments of the present disclosure, the RNA (or mRNA respectively) contains one or more modifications, e.g., in order to increase its stability and/or increase translation efficiency and/or decrease immunogenicity and/or decrease cytotoxicity. For example, in order to increase expression of the RNA (such as mRNA), it may be modified within the coding region, i.e., the sequence encoding the expressed peptide or protein, preferably without altering the sequence of the expressed peptide or protein. Such modifications are described, for example, in WO 2007/036366 and PCT/EP2019/056502, and include the following: a 5'-cap structure; an extension or truncation of the naturally occurring poly( A) tail; an
alteration of the 5'- and/or 3'-untranslated regions (UTR) such as introduction of a UTR which is not related to the coding region of said RNA; the replacement of one or more naturally occurring nucleotides with synthetic nucleotides (examples of modified ribonucleotides are given below and include, without limitation, 5-methylcytidine, pseudouridine (Ψ), Nl-methyl-pseudouridine (m1Ψ) or 5-methyl-uridine (m5U)); and codon optimization (e.g., to alter, preferably increase, the GC content of the RNA). The term "modification" in the context of modified RNA (such as modified mRNA) according to the present disclosure preferably relates to any modification of an RNA (such as mRNA) which is not naturally present in said RNA (such as mRNA).

In some embodiments, a 5' cap includes a Cap-0, a Cap-1, or Cap-2.

**3' UTR:** In some embodiments, an RNA disclosed herein comprises a 3'-UTR. A 3'-UTR, if present, is located at the 3' end, downstream of the termination codon of a protein-encoding region, but the term "3'-UTR" does preferably not include the poly(A) sequence. Thus, the 3'UTR is upstream of the poly(A) sequence (if present), *e.g*. directly adjacent to the poly( A) sequence.

**Poly A:** In some embodiments, an RNA disclosed herein comprises a polyadenylate (Poly A) sequence, *e.g.,* as described herein. In some embodiments, a Poly A sequence is situated downstream of a 3'-UTR, *e.g.,* adjacent to a 3'-UTR.

As used herein, the term "poly(A) sequence" or "poly-A tail" refers to an uninterrupted or interrupted sequence of adenylate residues which is typically located at the 3'-end of an RNA polynucleotide. Poly( A) sequences are known to those of skill in the art and may follow the 3 ' - UTR in the RN As described herein. An uninterrupted poly( A) sequence is characterized by consecutive adenylate residues. In nature, an uninterrupted poly(A) sequence is typical. RNAs disclosed herein can have a poly( A) sequence attached to the free 3'-end of the RNA by a template-independent RNA polymerase after transcription or a poly( A) sequence encoded by DNA and transcribed by a template-dependent RNA polymerase.

It has been demonstrated that in some embodiments, a poly( A) sequence of about 120 A nucleotides has a beneficial influence on the levels of RNA in transfected eukaryotic cells, as well as on the levels of protein that is translated from an open reading frame that is present upstream (5') of the poly( A) sequence *(*Holtkamp et al., 2006, Blood, vol. 108, pp. 4009-4017). The poly( A) sequence may be of any length. In some embodiments, a poly( A) sequence comprises, essentially consists of, or consists of at least 20, at least 30, at least 40, at least 80, or at least 100 and up to 500, up to 400, up to 300, up to 200, or up to 150 A nucleotides, and, in particular, about 120 A nucleotides. In this context, "essentially consists of' means that most nucleotides in the poly( A) sequence, typically at least 75%, at least 80%, at least 85%, at least 90%, at least 95%, at least 96%, at least 97%, at least 98%, or at least 99% by number of nucleotides in the poly(A) sequence are A nucleotides, but permits that remaining nucleotides are nucleotides other than A nucleotides, such as U nucleotides (uridylate ), G nucleotides (guanylate), or C nucleotides (cytidylate). In this context, "consists of' means that all nucleotides in the poly( A) sequence, *i.e.,* 100% by number of nucleotides in the poly( A) sequence, are A nucleotides. The term "A nucleotide" or "A" refers to adenylate.

**Nucleic acid containing particles** / **Nanovesicles:** Nucleic acids described herein such as mRNA encoding HMGA1 or HMGA2 or parts thereof as defined herein may be administered formulated as particles. In the context of the present disclosure, the term "particle" relates to a structured entity formed by molecules or molecule complexes. In some embodiments, the term "particle" relates to a micro- and preferably nano-sized structure, such as a micro- or nano-sized compact structure dispersed in a medium. Independent of their size, such particles are also interchangeably referred to as "nanoparticles" or "nanovesicles" herein as far these particles/vesicles have an average diameter suitable for parenteral administration. In some embodiments, a particle is a nucleic acid containing particle such as a nanovesicle comprising DNA, RNA or a mixture thereof, preferably a mRNA encoding HMGA1 or HMGA2 or parts thereof as defined herein. In some embodiments, nucleic acid containing particles include lipid nanoparticles, lipoplex, polyplexes (PLX), lipidated polyplexes (LPLX), liposomes, or polysaccharide nanoparticles. Such particles are known in the art to deliver an active agent. See, *e.g.,* Lachelt, Ulrich, and Ernst Wagner. "Nucleic acid therapeutics using polyplexes: a journey of 50 years (and beyond)" Chemical reviews 115.19 (2015): 11043-11078; Plucinski, Alexander, Zan Lyu, and Bernhard VKJ Schmidt, "Polysaccharide nanoparticles: from fabrication to applications." Journal of Materials Chemistry B (2021); and Tenchov, Rumiana, et al. "Lipid Nanoparticles- From Liposomes to mRNA Vaccine Delivery, a Landscape of Research Diversity and Advancement," ACS nano 15.11 (2021): 16982-17015, the contents of each of which are hereby incorporated by reference herein in their entirety.

In some embodiments, electrostatic interactions between positively charged molecules such as polymers and lipids and negatively charged nucleic acid are involved in particle formation. This results in complexation and spontaneous formation of nucleic acid particles. In some embodiments, a nucleic acid particle is a nanoparticle referred partially interchangeably to also as nanovesicle.

A "nucleic acid particle" can be used to deliver nucleic acid to a target site of interest *(e.g.,* cell, tissue, organ, and the like). A nucleic acid particle may be formed from at least one cationic or cationically ionizable lipid or lipid-like material, at least one cationic polymer such as protamine, or a mixture thereof and nucleic acid. Nucleic acid particles include lipid nanoparticle (LNP)-based and lipoplex (LPX)-based formulations.

As those skilled in the art are aware term "protamine" is often used to refer to any of various strongly basic proteins of relatively low molecular weight that are rich in arginine and are found associated especially with DNA in place of somatic hi stones in the sperm cells of various animals (as fish). In particular, the term "protamine" is often used to refer to proteins found in fish sperm that are strongly basic, are soluble in water, are not coagulated by heat, and yield chiefly arginine upon hydrolysis. In purified form, they are used in a long-acting formulation of insulin and to neutralize the anticoagulant effects of heparin. In some embodiments, the term "protamine" as used herein is refers to a protamine amino acid sequence obtained or derived from natural or biological sources, including fragments thereof and/or multimeric forms of said amino acid sequence or fragment thereof, as well as (synthesized) polypeptides which are artificial and specifically designed for specific purposes and cannot be isolated from native or biological sources.

Without intending to be bound by any theory, it is believed that the cationic or cationically ionizable lipid or lipid-like material and/or the cationic polymer combine together with the nucleic acid to form aggregates, and this aggregation results in colloidally stable particles.

In some embodiments, particles described herein further comprise at least one lipid or lipid-like material other than a cationic or cationically ionizable lipid or lipid-like material, at least one polymer other than a cationic polymer, or a mixture thereof. In some embodiments, nucleic acid particles comprise more than one type of nucleic acid molecules, where the molecular parameters of the nucleic acid molecules may be similar or different from each other, like with respect to molar mass or fundamental structural elements such as molecular architecture, capping, coding regions or other features. Nucleic acid particles described herein may have an average diameter that in some embodiments ranges from about 30 nm to about 1000 nm, from about 50 nm to about 800 nm, from about 70 nm to about 600 nm, from about 90 nm to about 400 nm, or from about 100 nm to about 300 nm. In some embodiments, nucleic acid particles described herein may have an average diameter ranging from about 50 nm to about 200 nm or from about 50 nm to about 150 nm, or from about 50 nm to about 100 nm.

Nucleic acid particles described herein may exhibit a polydispersity index less than about 0.5, less than about 0.4, less than about 0.3, or about 0.2 or less. By way of example, the nucleic acid particles can exhibit a polydispersity index in a range of about 0.1 to about 0.3 or about 0.2 to about 0.3.

With respect to RNA lipid particles, the *NIP* ratio gives the ratio of the nitrogen groups in the lipid to the number of phosphate groups in the RNA It is correlated to the charge ratio, as the nitrogen atoms (depending on the pH) are usually positively charged and the phosphate groups are negatively charged. The *NIP* ratio, where a charge equilibrium exists, depends on the pH. Lipid formulations are frequently formed at *NIP* ratios larger than four up to twelve, because positively charged nanoparticles are considered favorable for transfection. In that case, RNA is considered to be completely bound to nanoparticles.

Nucleic acid particles described herein can be prepared using a wide range of methods that may involve obtaining a colloid from at least one cationic or cationically ionizable lipid or lipid-like material and/or at least one cationic polymer and mixing the colloid with nucleic acid to obtain nucleic acid particles.

The term "colloid" as used herein relates to a type of homogeneous mixture in which dispersed particles do not settle out. The insoluble particles in the mixture are microscopic, with particle sizes between 1 and 1000 nanometers. The mixture may be termed a colloid or a colloidal suspension. Sometimes the term "colloid" only refers to the particles in the mixture and not the entire suspension.

For the preparation of colloids comprising at least one cationic or cationically ionizable lipid or lipid-like material and/or at least one cationic polymer methods are applicable herein that are conventionally used for preparing liposomal vesicles and are appropriately adapted. The most commonly used methods for preparing liposomal vesicles share the following fundamental stages: (i) lipids dissolution in organic solvents, (ii) drying of the resultant solution, and (iii) hydration of dried lipid (using various aqueous media).

In the film hydration method, lipids are firstly dissolved in a suitable organic solvent, and dried down to yield a thin film at the bottom of the flask. The obtained lipid film is hydrated using an appropriate aqueous medium to produce a liposomal dispersion. Furthermore, an additional downsizing step may be included. Reverse phase evaporation is an alternative method to the film hydration for preparing liposomal vesicles that involves formation of a water-in-oil emulsion between an aqueous phase and an organic phase containing lipids. A brief sonication of this mixture is required for system homogenization. The removal of the organic phase under reduced pressure yields a milky gel that turns subsequently into a liposomal suspension. The term "ethanol injection technique" refers to a process, in which an ethanol solution comprising lipids is rapidly injected into an aqueous solution through a needle. This action disperses the lipids throughout the solution and promotes lipid structure formation, for example lipid vesicle formation such as liposome formation. Generally, the RNA lipoplex particles described herein are obtainable by adding RNA to a colloidal liposome dispersion. Using the ethanol injection technique, such colloidal liposome dispersion is, in some embodiments, formed as follows: an ethanol solution comprising lipids, such as cationic lipids and additional lipids, is injected into an aqueous solution under stirring. In some embodiments, the RNA lipoplex particles described herein are obtainable without a step of extrusion. The term "extruding" or "extrusion" refers to the creation of particles having a fixed, cross-sectional profile. In particular, it refers to the downsizing of a particle, whereby the particle is forced through filters with defined pores.

Other methods having organic solvent free characteristics may also be used according to the present disclosure for preparing a colloid.

LNPs typically comprise four components: ionizable cationic lipids, neutral lipids such as phospholipids, a steroid such as cholesterol, and a polymer conjugated lipid such as polyethylene glycol (PEG)-lipids. Each component is responsible for payload protection, and enables effective intracellular delivery. LNPs may be prepared by mixing lipids dissolved in ethanol rapidly with nucleic acid in an aqueous buffer.

The term "average diameter" refers to the mean hydrodynamic diameter of particles as measured by dynamic laser light scattering (DLS) with data analysis using the so-called cumulant algorithm, which provides as results the so-called Zaverage with the dimension of a length, and the polydispersity index (PI), which is dimensionless (Koppel, D., J. Chem. Phys. 57, 1972, pp 4814-4820, ISO 13321). Here "average diameter", "diameter" or "size" for particles is used synonymously with this value of the Zaverage.

The "polydispersity index" is preferably calculated based on dynamic light scattering measurements by the so-called cumulant analysis as mentioned in the definition of the "average diameter". Under certain prerequisites, it can be taken as a measure of the size distribution of an ensemble of nanoparticles/nanovisicles.

Different types of nucleic acid containing particles have been described previously to be suitable for delivery of nucleic acid in particulate form (e.g., Kaczmarek, J. C. et al. 2017, Genome Medicine 9, 60). For non-viral nucleic acid delivery vehicles, nanoparticle encapsulation of nucleic acid physically protects nucleic acid from degradation and, depending on the specific chemistry, can aid in cellular uptake and endosomal escape.

The present disclosure describes particles comprising nucleic acid, at least one cationic or cationically ionizable lipid or lipid-like material, and/or at least one cationic polymer which associate with nucleic acid to form nucleic acid particles and compositions comprising such particles. The nucleic acid particles may comprise nucleic acid which is complexed in different forms by non-covalent interactions to the particle. The particles described herein are not viral particles, in particular infectious viral particles, *i.e.,* they are not able to virally infect cells. Suitable cationic or cationically ionizable lipids or lipid-like materials and cationic polymers are those that form nucleic acid particles and are included by the term "particle forming components" or "particle forming agents". The term "particle forming components" or "particle forming agents" relates to any components which associate with nucleic acid to form nucleic acid particles. Such components include any component which can be part of nucleic acid particles.

**Cationic polymeric materials *(e.g.,* polymers):** Given their high degree of chemical flexibility, polymeric materials are commonly used for nanoparticle-based delivery. Typically, cationic materials are used to electrostatically condense the negatively charged nucleic acid into nanoparticles. These positively charged groups often consist of amines that change their state of protonation in the pH range between 5. 5 and 7.5, thought to lead to an ion imbalance that results in endosomal rupture. Polymers such as poly-L-lysine, polyamidoamine, protamine and polyethyleneimine, as well as naturally occurring polymers such as chitosan have all been applied to nucleic acid delivery and are suitable as cationic materials useful in some embodiments herein. In addition, some investigators have synthesized polymeric materials specifically for nucleic acid delivery. Poly(β-amino esters), in particular, have gained widespread use in nucleic acid delivery owing to their ease of synthesis and biodegradability. In some embodiments, such synthetic materials may be suitable for use as cationic materials herein. A "polymeric material", as used herein, is given its ordinary meaning, *i.e.,* a molecular structure comprising one or more repeat units (monomers), connected by covalent bonds. In some embodiments, such repeat units can all be identical; alternatively, in some cases, there can be more than one type of repeat unit present within the polymeric material. In some cases, a polymeric material is biologically derived, *e.g.,* a biopolymer such as a protein. In some cases, additional moieties can also be present in the polymeric material, for example targeting moieties such as those described herein.

Those skilled in the art are aware that, when more than one type of repeat unit is present within a polymer (or polymeric moiety), then the polymer (or polymeric moiety) is said to be a "copolymer." In some embodiments, a polymer (or polymeric moiety) utilized in accordance with the present disclosure may be a copolymer. Repeat units forming the copolymer can be arranged in any fashion. For example, in some embodiments, repeat units can be arranged in a random order; alternatively or additionally, in some embodiments, repeat units may be arranged in an alternating order, or as a "block" copolymer, *i.e.,* comprising one or more regions each comprising a first repeat unit *(e.g.,* a first block), and one or more regions each comprising a second repeat unit *(e.g.,* a second block), etc. Block copolymers can have two (a deblock copolymer), three (a triblock copolymer), or more numbers of distinct blocks.

In certain embodiments, a polymeric material for use in accordance with the present disclosure is biocompatible. Biocompatible materials are those that typically do not result in significant cell death at moderate concentrations. In certain embodiments, a biocompatible material is biodegradable, *i.e.,* is able to degrade, chemically and/or biologically, within a physiological environment, such as within the body.

As used herein, the term "pharmaceutical composition" refers to a mixture containing a therapeutic agent (*e.g.,* a mRNA or a nanovesicle described herein), optionally in combination with one or more pharmaceutically acceptable excipients, diluents, and/or carriers. The pharmaceutical composition is, for example, formulated for administration to a subject, such as a mammal, *e.g.,* a human, in order to prevent, treat or control a particular disease or condition affecting, or that may affect, the subject (*e.g.,* arthrose, arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis).

Cationic materials *(e.g.,* polymeric materials, including polycationic polymers) contemplated for use herein include those which are able to electrostatically bind nucleic acid. In some embodiments, cationic polymeric materials contemplated for use herein include any cationic polymeric materials with which nucleic acid can be associated, *e.g*. by forming complexes with the nucleic acid or forming vesicles in which the nucleic acid is enclosed or encapsulated.In some embodiments, particles described herein may comprise polymers other than cationic polymers, *e.g.,* non-cationic polymeric materials and/or anionic polymeric materials. Collectively, anionic and neutral polymeric materials are referred to herein as non-cationic polymeric materials.

**Lipid and lipid-like material:** The terms "lipid" and "lipid-like material" are used herein to refer to molecules which comprise one or more hydrophobic moieties or groups and optionally also one or more hydrophilic moieties or groups. Molecules comprising hydrophobic moieties and hydrophilic moieties are also frequently denoted as amphiphiles. Lipids are usually poorly soluble in water.

In an aqueous environment, the amphiphilic nature allows the molecules to self-assemble into organized structures and different phases. One of those phases consists of lipid bilayers, as they are present in vesicles, multilamellar/unilamellar liposomes, or membranes in an aqueous environment. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). In some embodiments, hydrophilic groups may comprise polar and/or charged groups and include carbohydrates, phosphate, carboxylic, sulfate, amino, sulfuydryl, nitro, hydroxyl, and other like groups. As used herein, the term "amphiphilic" refers to a molecule having both a polar portion and a non-polar portion. Often, an amphiphilic compound has a polar head attached to a long hydrophobic tail. In some embodiments, the polar portion is soluble in water, while the non-polar portion is insoluble in water. In addition, the polar portion may have either a formal positive charge, or a formal negative charge. Alternatively, the polar portion may have both a formal positive and a negative charge, and be a zwitterion or inner salt. For purposes of the disclosure, the amphiphilic compound can be, but is not limited to, one or a plurality of natural or nonnatural lipids and lipid-like compounds. The term "lipid-like material", "lipid-like compound" or "lipid-like molecule" relates to substances that structurally and/or functionally relate to lipids but may not be considered as lipids in a strict sense. For example, the term includes compounds that are able to form amphiphilic layers as they are present in vesicles, multilamellar/unilamellar liposomes, or membranes in an aqueous environment and includes surfactants, or synthesized compounds with both hydrophilic and hydrophobic moieties. Generally speaking, the term refers to molecules, which comprise hydrophilic and hydrophobic moieties with different structural organization, which may or may not be similar to that of lipids. As used herein, the term "lipid" is to be construed to cover both lipids and lipid-like materials unless otherwise indicated herein or clearly contradicted by context.

Specific examples of amphiphilic compounds that may be included in an amphiphilic layer include, but are not limited to, phospholipids, aminolipids and sphingolipids.

In certain embodiments, the amphiphilic compound is a lipid. The term "lipid" refers to a group of organic compounds that are characterized by being insoluble in water, but soluble in many organic solvents. Generally, lipids may be divided into eight categories: fatty acids, glycerolipids, glycerophospholipids, sphingolipids, saccharolipids, polyketides (derived from condensation of ketoacyl subunits), sterol lipids and prenol lipids (derived from condensation of isoprene subunits). Although the term "lipid" is sometimes used as a synonym for fats, fats are a subgroup of lipids called triglycerides. Lipids also encompass molecules such as fatty acids and their derivatives (including tri-, di-, monoglycerides, and phospholipids), as well as sterolcontaining metabolites such as cholesterol.

**Cationic or cationically ionizable lipids or lipid-like materials:** In some embodiments, nucleic acid particles described and/or utilized in accordance with the present disclosure may comprise at least one cationic or cationically ionizable lipid or lipid-like material as particle forming agent. Cationic or cationically ionizable lipids or lipid-like materials contemplated for use herein include any cationic or cationically ionizable lipids or lipid-like materials which are able to electrostatically bind nucleic acid. In some embodiments, cationic or cationically ionizable lipids or lipid-like materials contemplated for use herein can be associated with nucleic acid, *e.g.* by forming complexes with the nucleic acid or forming vesicles in which the nucleic acid is enclosed or encapsulated.

As used herein, a "cationic lipid" or "cationic lipid-like material" refers to a lipid or lipid-like material having a net positive charge. Cationic lipids or lipid-like materials bind negatively charged nucleic acid by electrostatic interaction. Generally, cationic lipids possess a lipophilic moiety, such as a sterol, an acyl chain, a diacyl or more acyl chains, and the head group of the lipid typically carries the positive charge. In certain embodiments, a cationic lipid or lipid-like material has a net positive charge only at certain pH, in particular acidic pH, while it has preferably no net positive charge, preferably has no charge, *i.e.,* it is neutral, at a different, preferably higher pH such as physiological pH. This ionizable behavior is thought to enhance efficacy through helping with endosomal escape and reducing toxicity as compared with particles that remain cationic at physiological pH. For purposes of the present disclosure, such "cationically ionizable" lipids or lipid-like materials are comprised by the term "cationic lipid or lipid-like material" unless contradicted by the circumstances.

**Additional lipids or lipid-like materials:** In some embodiments, particles described herein comprise *(e.g.,* in addition to a cationic lipid such as ALC315), one or more lipids or lipid-like materials other than cationic or cationically ionizable lipids or lipid-like materials, *e.g.,* non-cationic lipids or lipid-like materials (including non-cationically ionizable lipids or lipid-like materials). Collectively, anionic and neutral lipids or lipid-like materials are referred to herein as non-cationic lipids or lipid-like materials. Optimizing the formulation of nucleic acid particles by addition of other hydrophobic moieties, such as cholesterol and lipids, in addition to an ionizable/cationic lipid or lipid-like material may enhance particle stability and efficacy of nucleic acid delivery. An additional lipid or lipid-like material may be incorporated which may or may not affect the overall charge of the nucleic acid particles. In certain embodiments, the additional lipid or lipid-like material is a non-cationic lipid or lipid-like material. The non-cationic lipid may comprise, *e.g.,* one or more anionic lipids and/or neutral lipids. As used herein, an "anionic lipid" refers to any lipid that is negatively charged at a selected pH. As used herein, a "neutral lipid" refers to any of a number of lipid species that exist either in an uncharged or neutral zwitterionic form at a selected pH. In preferred embodiments, the additional lipid comprises one of the following neutral lipid components: (1) a phospholipid, (2) cholesterol or a derivative thereof; or (3) a mixture of a phospholipid and cholesterol or a derivative thereof. Examples of cholesterol derivatives include, but are not limited to, cholestanol, cholestanone, cholestenone, coprostanol, cholesteryl-2'-hydroxyethyl ether, cholesteryl-4'- hydroxybutyl ether, tocopherol and derivatives thereof, and mixtures thereof. Specific phospholipids that can be used include, but are not limited to, phosphatidylcholines, phosphatidylethanolamines, phosphatidylglycerols, phosphatidic acids, phosphatidylserines or sphingomyelin.

In certain embodiments, the nucleic acid particles include both a cationic lipid and an additional lipid. In some embodiments, particles described herein include a polymer conjugated lipid such as a pegylated lipid. The term "pegylated lipid" refers to a molecule comprising both a lipid portion and a polyethylene glycol portion. Pegylated lipids are known in the art. In some embodiments, a pegylated lipid is ALC-0159, also referred to herein as (2-[(polyethylene glycol)-2000]-N,N-ditetradecylacetamide).

**Lipoplex Particles:** In certain embodiments of the present disclosure, an RNA may be present in RNA lipoplex particles. In the context of the present disclosure, the term "RNA lipoplex particle" relates to a particle that contains lipid, in particular cationic lipid, and RNA. Electrostatic interactions between positively charged liposomes and negatively charged RNA results in complexation and spontaneous formation of RNA lipoplex particles. Positively charged liposomes may be generally synthesized using a cationic lipid, such as DOTMA, and additional lipids, such as DOPE. In some embodiments, a RNA lipoplex particle is a nanoparticle. In certain embodiments, the RNA lipoplex particles include both a cationic lipid and an additional lipid. In an exemplary embodiment, the cationic lipid is DOTMA and the additional lipid is DOPE.

**Lipid nanoparticles (LNPs):** In some embodiments, nucleic acid such as RNA described herein is administered in the form of lipid nanoparticles (LNPs). The LNP may comprise any lipid capable of forming a particle to which the one or more nucleic acid molecules are attached, or in which the one or more nucleic acid molecules are encapsulated. In some embodiments, the LNP comprises one or more cationic lipids, and one or more stabilizing lipids. Stabilizing lipids include neutral lipids and pegylated lipids. In some embodiments, the LNP comprises a cationic lipid, a neutral lipid, a steroid, a polymer conjugated lipid; and the RNA, encapsulated within or associated with the lipid nanoparticle.

In some embodiments, a pharmaceutical composition comprises an RNA polynucleotide, in particular a mRNA encoding HMGA1 or HMGA2 or parts thereof disclosed herein formulated as a particle. In some embodiments, a particle is or comprises a lipid nanoparticle (LNP) or a lipoplex (LPX) particle. In some embodiments, an RNA polynucleotide disclosed herein may be administered in a pharmaceutical composition or a medicament and may be administered in the form of any suitable pharmaceutical composition.

In some embodiments, an RNA polynucleotide disclosed herein may be administered in a pharmaceutical composition which may comprise a pharmaceutically acceptable carrier and may optionally comprise one or more adjuvants, stabilizers etc. In some embodiments, a pharmaceutical composition is for therapeutic or prophylactic treatments.

The pharmaceutical compositions according to the present disclosure are generally applied in a "pharmaceutically effective amount" and in "a pharmaceutically acceptable preparation". The term "pharmaceutically acceptable" refers to the non-toxicity of a material which does not interact with the action of the active component of the pharmaceutical composition. The term "pharmaceutically effective amount" or "therapeutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the compositions described herein may depend on various of such parameters. In the case that a reaction in a patient is insufficient with an initial dose, higher doses (or effectively higher doses achieved by a different, more localized route of administration) may be used. In some embodiments, a pharmaceutical composition disclosed herein may contain salts, buffers, preservatives, and optionally other therapeutic agents. In some embodiments, a pharmaceutical composition disclosed herein comprises one or more pharmaceutically acceptable carriers, diluents and/or excipients. Suitable preservatives for use in a pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants. The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In some embodiments, the pharmaceutical composition of the present disclosure includes isotonic saline.

As used herein, the term "pharmaceutically acceptable" refers to those compounds, materials, compositions, and/or dosage forms which are, within the scope of sound medical judgment, suitable for use in contact with the tissues a subject, such as an animal, *e.g.,* a mammal (e.g., a human) without excessive toxicity, irritation, allergic response, or other problem or complication, commensurate with a reasonable benefit/risk ratio.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985). Pharmaceutical carriers, excipients or diluents can be selected with regard to the intended route of administration and standard pharmaceutical practice. In some embodiments, a pharmaceutical composition described herein may be administered intravenously, intraarterially, subcutaneously, intradermally or intramuscularly. In certain embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In a preferred embodiment, the pharmaceutical composition is formulated for intramuscular or intra-articular administration. In another embodiment, the pharmaceutical composition is formulated for systemic administration, e.g., for intravenous administration.

The term "between" as used herein include the endpoints.

"Percent (%) sequence identity" with respect to a reference polynucleotide or polypeptide sequence is defined as the percentage of nucleic acids or amino acids in a candidate sequence that are identical to the nucleic acids or amino acids in the reference polynucleotide or polypeptide sequence, after aligning the sequences and introducing gaps, if necessary, to achieve the maximum percent sequence identity. Alignment for purposes of determining percent nucleic acid or amino acid sequence identity can be achieved in various ways that are within the capabilities of one of skill in the art, for example, using publicly available computer software such as BLAST, BLAST-2, or Megalign software. Those skilled in the art can determine appropriate parameters for aligning sequences, including any algorithms needed to achieve maximal alignment over the full length of the sequences being compared. For example, percent sequence identity values may be generated using the sequence comparison computer program BLAST. As an illustration, the percent sequence identity of a given nucleic acid or amino acid sequence, A, to, with, or against a given nucleic acid or amino acid sequence, B, (which can alternatively be phrased as a given nucleic acid or amino acid sequence, A that has a certain percent sequence identity to, with, or against a given nucleic acid or amino acid sequence, B) is calculated as follows:
100 multiplied by (the fraction X/Y)
where X is the number of nucleotides or amino acids scored as identical matches by a sequence alignment program (*e.g.,* BLAST) in that program's alignment of A and B, and where Y is the total number of nucleic acids in B. It will be appreciated that where the length of nucleic acid or amino acid sequence A is not equal to the length of nucleic acid or amino acid sequence B, the percent sequence identity of A to B will not equal the percent sequence identity of B to A.

As used herein, the terms "treat" or "treatment" refer to both therapeutic treatment and prophylactic or preventative measures, in which the object is to prevent or slow down (lessen) an undesired physiological change or disorder, such as the development of arthrose, arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis.

Beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable.

"Treatment" can also mean prolonging the symptom free period or a period with reduced symptoms of arthrose, arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis or any other disease associated with bone and/or cartilage loss experienced by a subject for at least 1, 2, 3, 4, 5, 6, 7, 8, 9, 10, 11, 12, 13, 14, 15, 16, 17, 18, 19, 20, or more months or years, *e.g.,* for the lifetime of the subject) as compared to expected symptom free period or a period with reduced symptoms of an individual with any of the listed diseases if not receiving treatment. Those in need of treatment include those already with the condition or disorder as well as those prone to have the condition or disorder or those in which the manifestation of the condition or disorder is to be prevented.

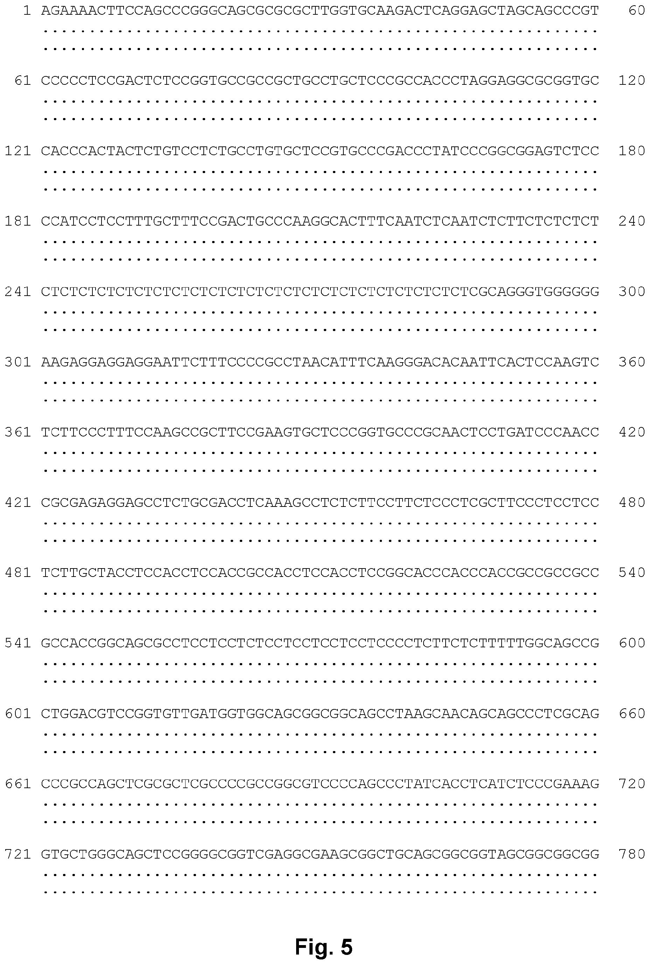

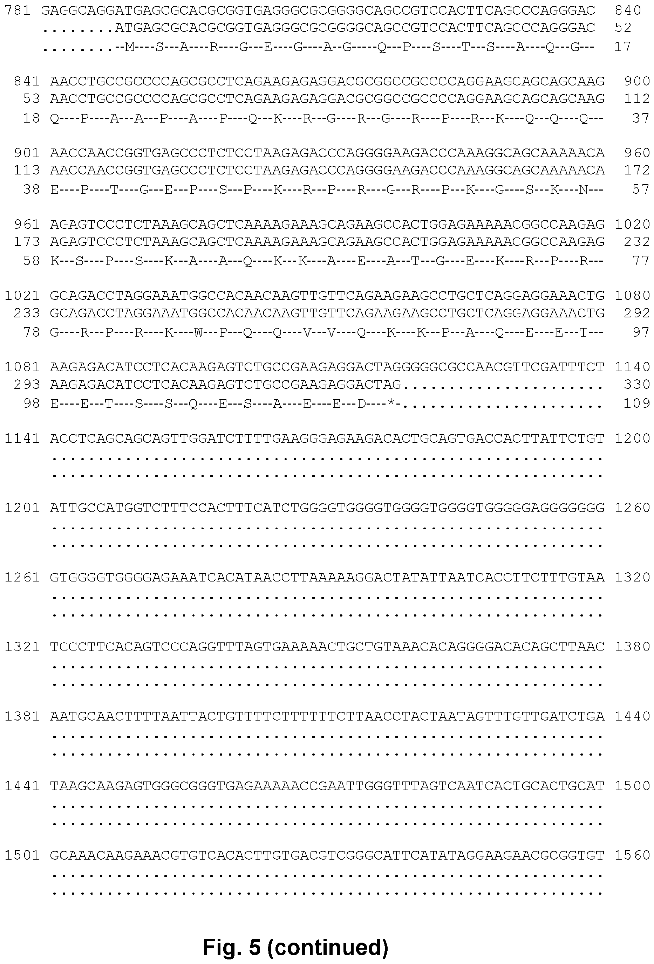

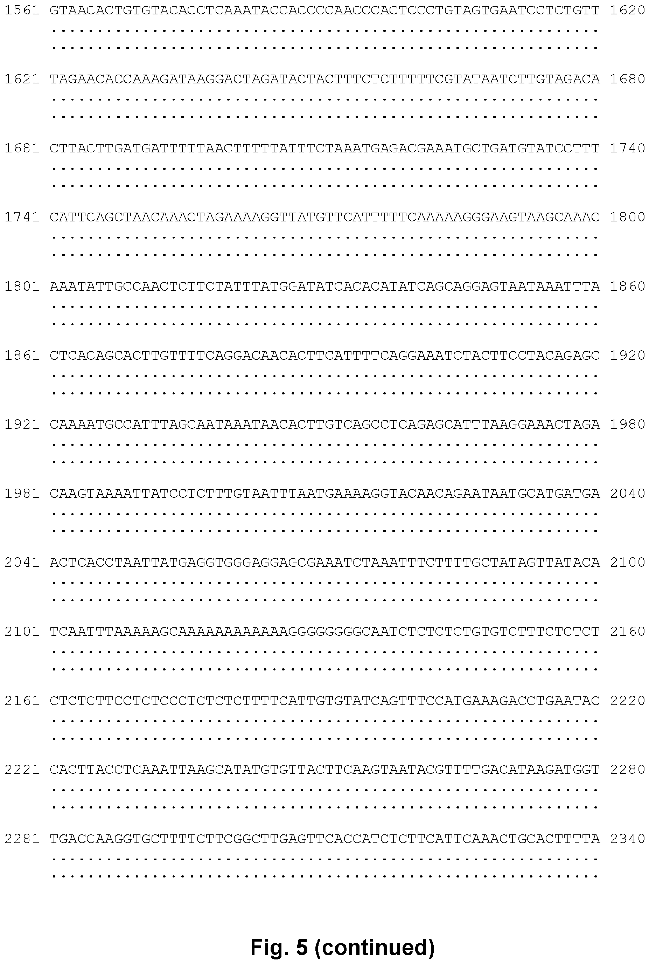

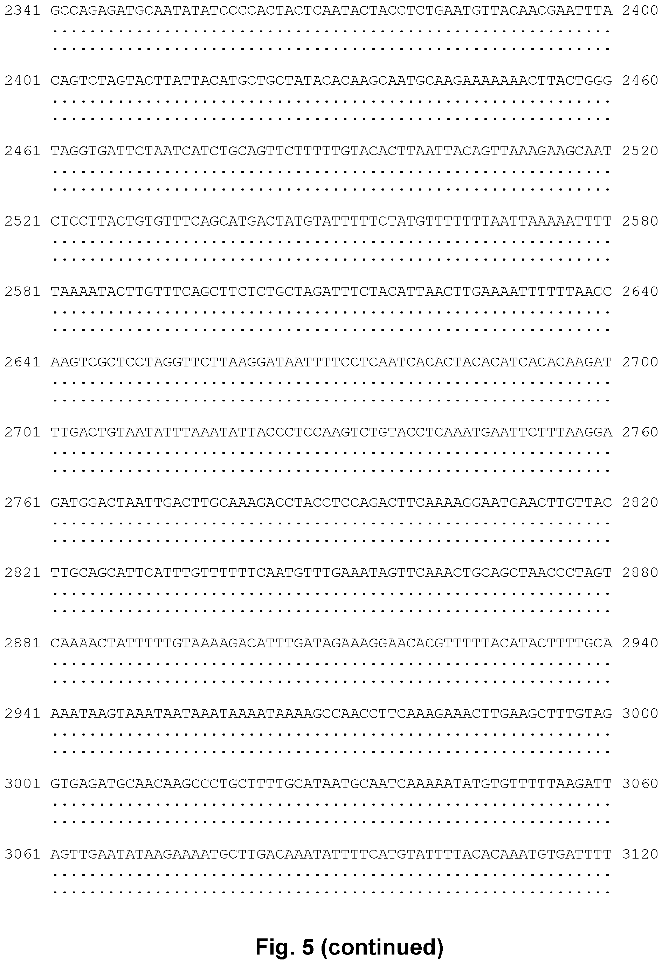

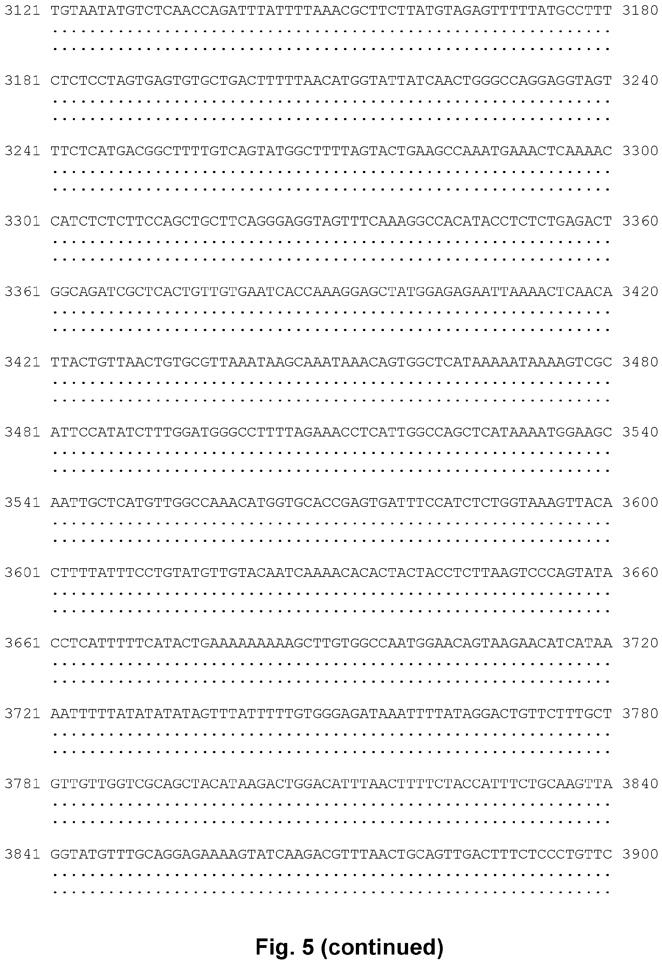

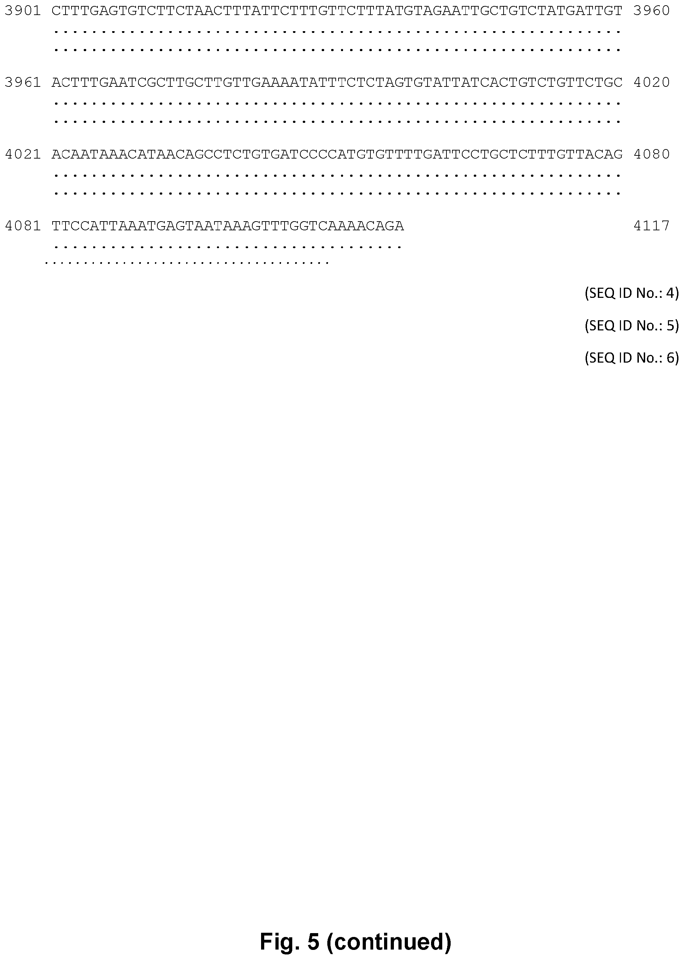

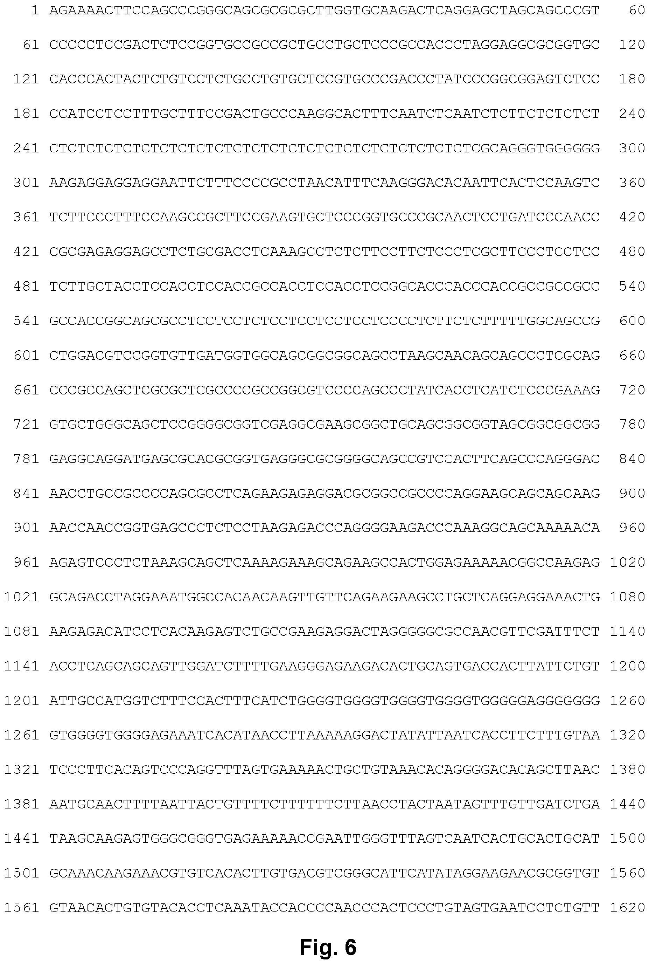

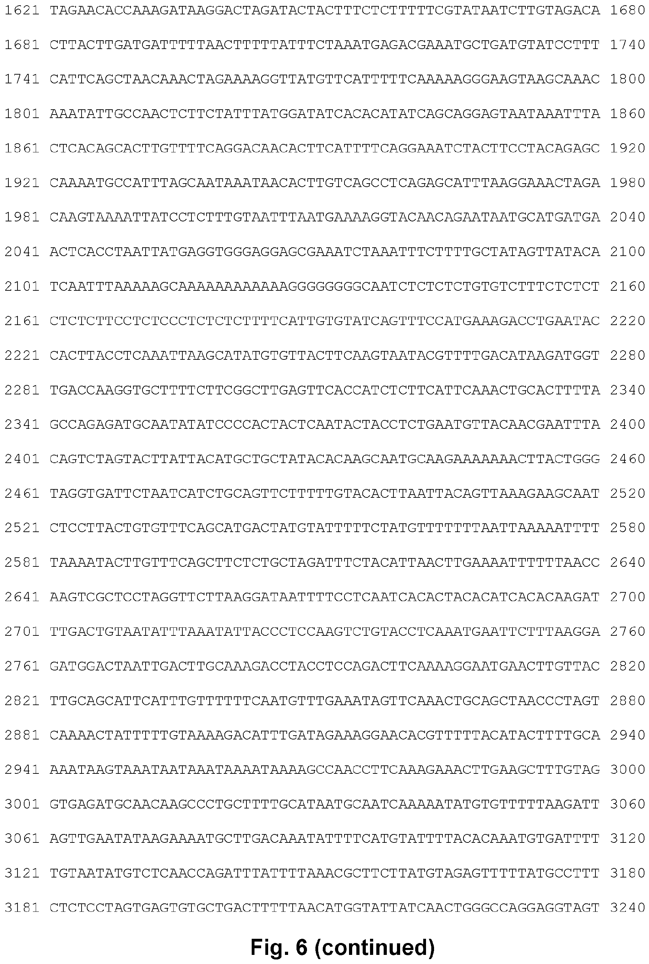

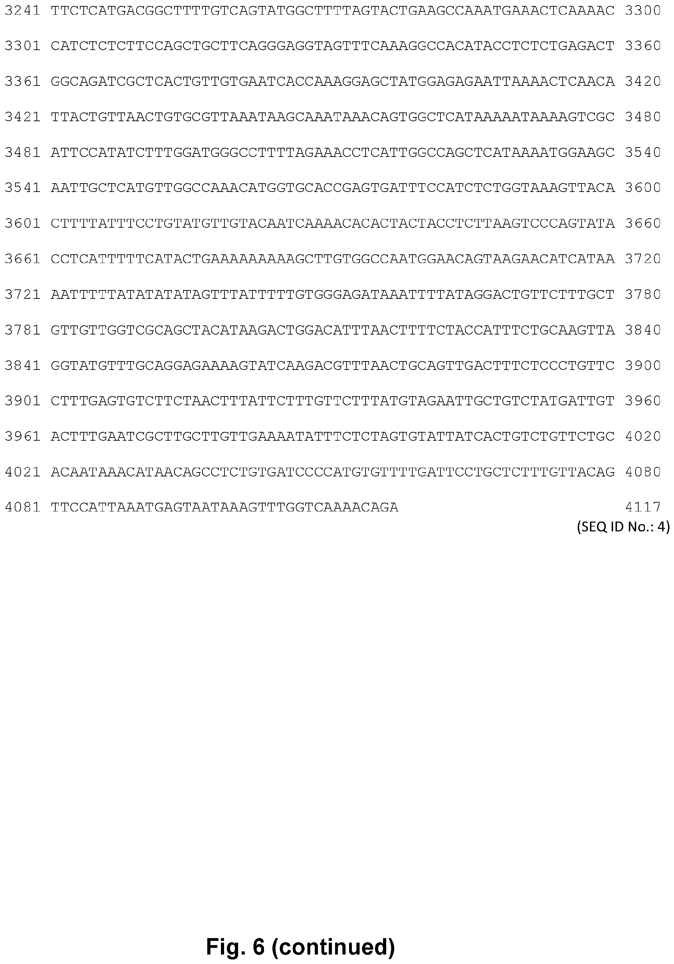

As discussed already above in general, the present invention provides an nucleic acid molecule, preferably a RNA, mostly preferred a mRNA molecule encoding HMGA2 or a part thereof for use in cell regeneration. If a part is encoded by the molecule, it is preferably the part of HMGA2 containing at least exons 1-3 of the cDNA with the SEQ ID No.: 4. Said sequence is shown in Figures 5 and 6.

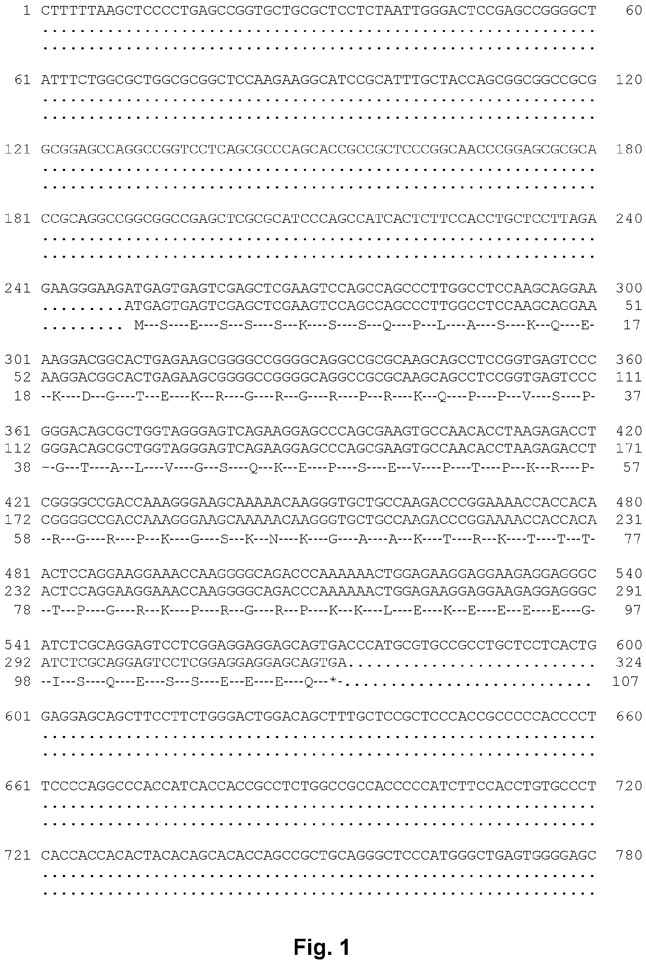

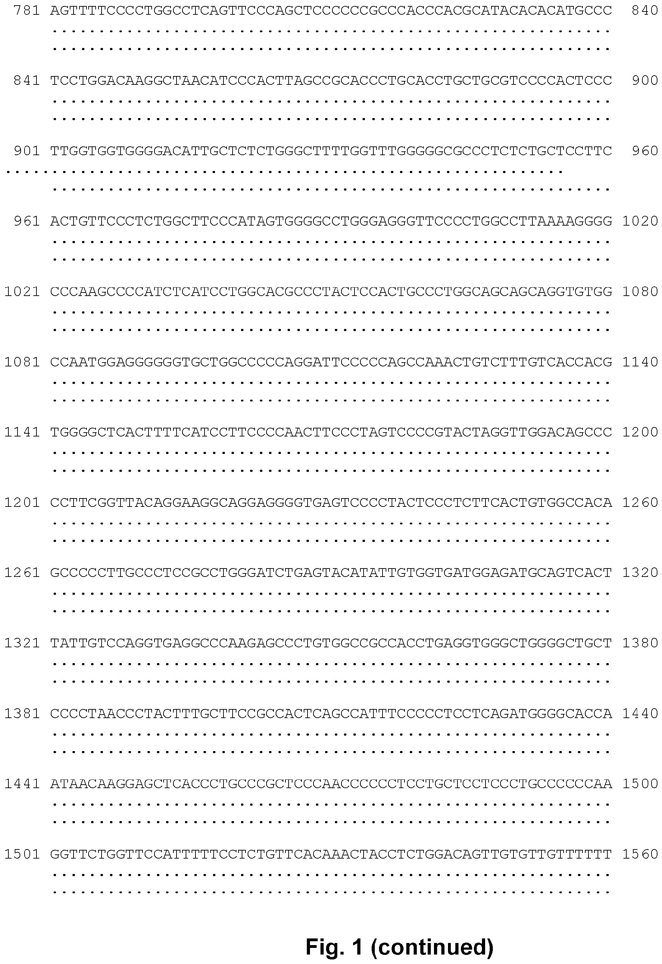

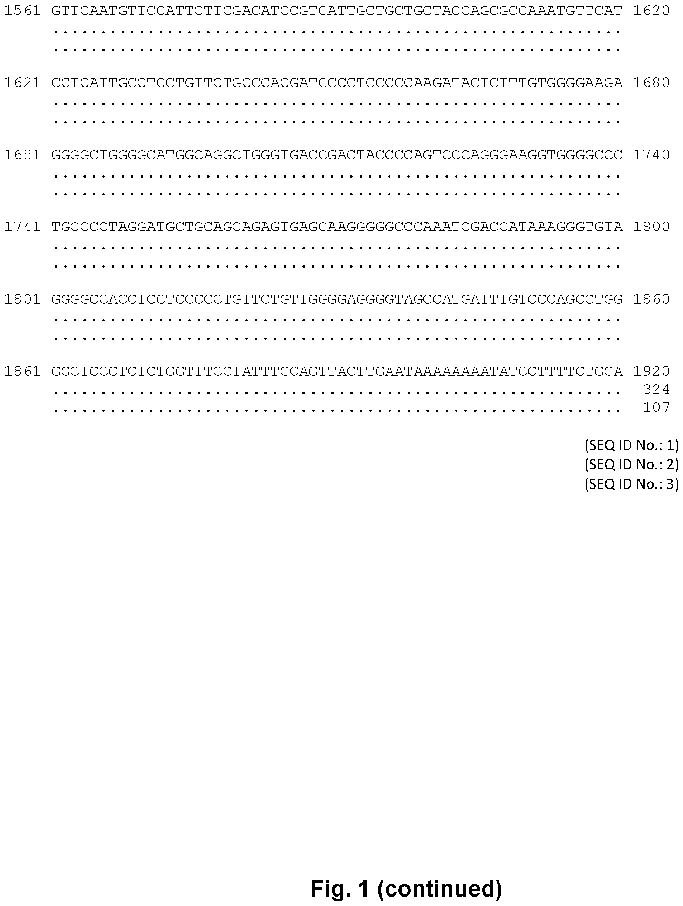

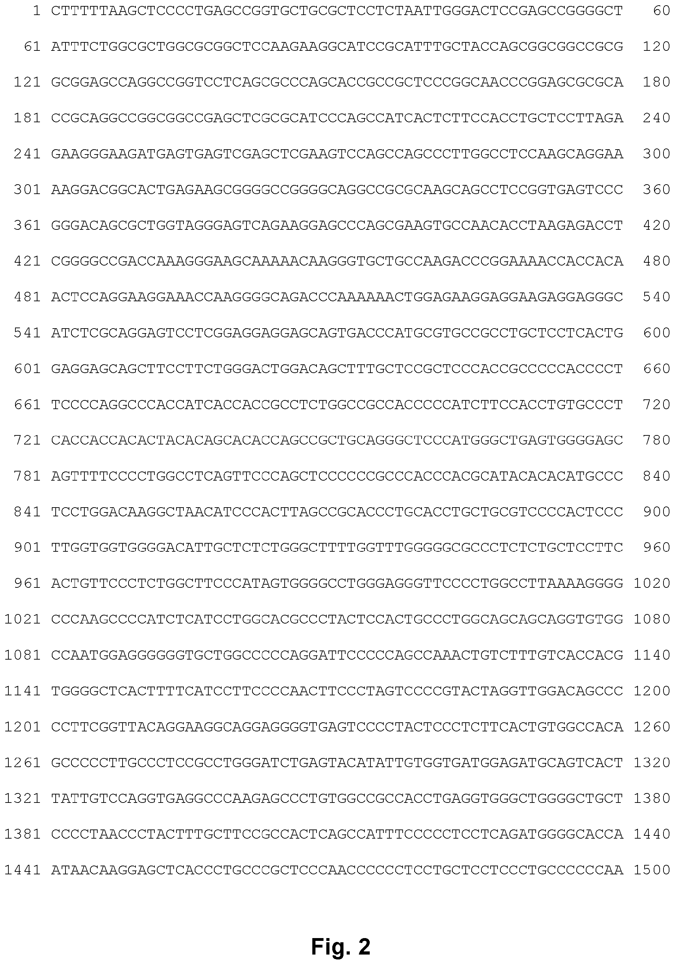

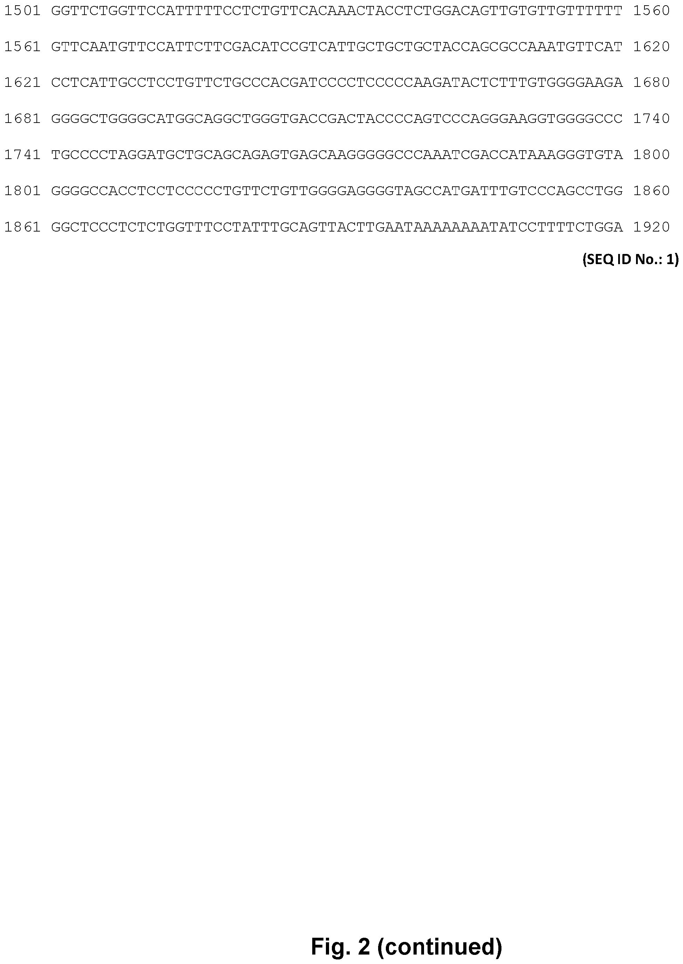

The present invention also provides a mRNA molecule encoding HMGA1 or part thereof for use in cell regeneration. If a part is encoded by the molecule, it is preferably the part of HMGA1 containing at least exons 1-3 of the cDNA with the SEQ ID NO.: 1. Said sequence is shown in Figures 1 and 2.

Preferably, the mRNA molecule for use in cell regeneration as described herein is provided, wherein the regenerated cells are selected from bone marrow stem cells and cartilage cells.

The term regeneration, as used herein preferably refers to a change in cells, preferably in bone marrow stem cells and cartilage cells, which can be defined as cells which dedifferentiated within the respective tissue into stem cells with accordingly regained potential for proliferation and differentiation and thus to produce differentiated tissue which has been lost, *e.g.,* due to aging or autoimmune diseases.

In addition, or alternatively, the mRNA molecule encoding HMGA1 or HMGA2, or part thereof containing at least exons 1-3 of the cDNA with the SEQ ID NO.:4 or SEQ ID NO.: 1 respectively is provided for use in cell rejuvenation.

The term rejuvenation, as used herein preferably refers to a change in cells, preferably in bone marrow stem cells and cartilage cells, which can be defined as increase the number of stem cells that are able to regain differentiated cells of the corresponding tissue.

Preferably, the nucleic acid molecule, preferably a RNA molecule and mostly preferred a mRNA molecule for use in cell regeneration and/or rejuvenation is provided as defined above, wherein the regenerated cells are selected from the group consisting of bone marrow stem cells including hematopoietic stem cells and mesenchymal stem cells, cartilage cells, cartilage stem cells, smooth muscle cells, smooth muscle stem cells, hairy cells and stem cells of the inner ear, soft tissue stem cells, and the rejuvenated cells are endometrium cells.

As indicated above, the regenerated cells in view of their regained stem cell properties can proliferate and differentiate to cells specific for different tissues, in particular to cells of the mesenchyme, cartilage, smooth muscles, hairy cells and cells of the inner ear, soft tissue cells and endometrium cells. Accordingly, in a preferred embodiment, the nucleic acid molecule, preferably RNA molecule and mostly preferred mRNA molecule as defined herein is provided for use in treatment of diseases related to cartilage loss.

Preferably, the nucleic acid molecule, preferably RNA molecule and mostly preferred mRNA molecule as defined herein is provided for use in treatment of diseases selected from the group consisting of arthrose, arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis.

As indicated above, N1-methylpseudouridine (m1Ψ) nucleoside modification has been shown as increasing biological stability of RNAs comprising it and thereby the durability of the encoded protein can be monitored longer compared to unmodified RNAs, see Karik6 et al. (2008) Molecular Therapy: the Journal of the American Society of Gene Therapy. Therefore, preferably the RNA molecule or mostly preferred the mRNA molecule for use as described herein is provided, wherein at least one uridine has been substituted by a modified uridine, such as a pseudouridine or a 1-methyl pseudouridine.

Preferably the RNA molecule or mRNA molecule as defined herein is provided, wherein at least 10 % uridine of the natural mRNA has been substituted by pseudouridine or N1-methyl-pseudouridine.

In a preferred embodiment between 20% and 30%, or between 30% and 40%, or between 40% and 50%, or between 50% and 60%, or between 60% and 70%, or between 70% and 80%, or between 80% and 90%of the uridines of the natural mRNA have been substituted by N1-methyl-pseudouridine. In one preferred embodiment the RNA molecule or mRNA molecule as defined herein is provided, wherein at least 90% of the uridines of the natural mRNA have been substituted by N1-methyl-pseudouridine.

As discussed above, the present invention fundamentally differs from the uses of RNA, respective RNA-Nanoparticles (or Nanovesicles comprising RNA) in vaccination, such as the COVID-19 vaccines in that it is not intended to induce immunogenicity against the expression product, i.e., polypeptide or protein encoded by the RNA, but it is rather intended to provide these expression products as a supplement to the subject to which the mRNA or Nanovesicle comprising it is administered to.

Accordingly, preferably the RNA molecule or preferably mRNA molecule for use according to the present invention is modified in such a way that a RNA molecule or preferably a mRNA molecule is provided, wherein uridines of the natural mRNA are substituted by residues with lower immunogenicity.

As shown in the Examples, disruption of all seven target sites of let-7-family miRNAs lead to a significantly enhanced expression of HMGA2 in the nuclei of the explants taken from fascia as well as from cartilageous tissue. Therefore, preferably at least one of the let-7 binding sites is been removed or mutated in the RNA molecule, or mostly preferred in the mRNA molecule for use as defined herein, so that the binding of the let-7-family miRNAs to the mRNA is reduced or abolished completely. In a preferred embodiment all let-7 binding sites are removed or mutated in the RNA molecule, or more preferably in the mRNA molecule for use according to the present invention.

As discussed above, packing RNAs or mRNAs into nanovesicles or similar combinations of RNAs/mRNAs with lipids, lipid-like material or cationic polymers, or combinations thereof improves their stability and also the delivery rates to a target site of interest (*e.g.,* cell, tissue, organ, and the like). One of the possibilities includes including the RNAs/mRNAs as defined herein into nanovesicles. Therefore, provided herein are also nanovesicles (NVs) containing at least one RNA/mRNA molecule or modified RNA/mRNA molecule as defined herein. In a preferred embodiment, the NV containing at least one mRNA molecule or modified mRNA molecule as defined herein is provided for use in cell regeneration and/or cell rejuvenation.

In addition, the NV is provided for use in treatment of diseases related to cartilage loss. Preferably, the NV as defined herein is provided for use in treatment of diseases selected from the group consisting of arthrosis (osteoarthritis), arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis.

Preferably, the nanovesicle(s) containing any of the RNAs/mRNAs or modified RNAs/mRNAs according to the present invention are provided, wherein the nanovesicle is selected from the group consisting of exosomes, artificial exosomes, lipid-based nanoparticles, liposomes, niosomes, ethosomes, and cell derived nanovesicles.

A liposome is a phospholipid forming a biomembrane or a lipid similar thereto and has the structure of a double membrane or a multiple membrane, the liposome being known as a self-closed colloidal particle having a size of tens of nanometers to hundreds of nanometers. Particularly, a self-closed colloidal particle prepared with a lipid of a nonionic surfactant is called a niosome. Ethosomes are a drug delivery system for enhancing skin absorption effect of liposome, and is a deformable formulation with more flexible membrane than liposome, which is able to easily pass through the narrow space between corneocytes (Dubey et al., 2007). Ethosomes are prepared by dissolving phospholipids in ethanol which is known as a skin permeation enhancer. Ethanol acts on polar heads of lipids to reduce surface tension, thereby reducing surface tension of lipid membranes in the stratum corneum and making the membrane of the vesicle itself flexible (Dubey et al., 2007). Due to these features, ethosomes are effective for delivery of active ingredients into the skin and are able to deliver active ingredients to the deeper layers of the skin (Adachi et al., 1995). Such characteristics of ethosomes are attributed to nanoliposomes containing ethanol. The content of ethanol in ethosomes is preferably about 20% or more. Ethanol is a substance that dehydrates and irritates the skin because ethanol evaporates along with the moisture of the skin when it is in contact with the skin. For this reason, as the ethanol content is higher, the skin irritation becomes more severe. In addition, since ethanol makes the lipid membrane flexible, there is a disadvantage that ethanol reduces encapsulation of the intended content, such as drugs or RNAs/mRNAs.

Exosomes are nanosized vesicles secreted by cells, and belong to extracellular vesicles (EVs). Exosomes are present in various biological fluids (such as amniotic fluid, urine, and blood), and are rich in proteins, lipids, mRNAs, and miRNAs (Valadi et al., 2007). Preparation of extracellular vesicles, e.g., exosomes, from ES cells has been described in the art (see e.g., Ke et al. (2021) Stem Cell Res. & Therap. 12:21). In addition, analogously or alternatively to the methods mentioned in section "Exosomes" above and described in WO99/03499, WO00/44389 and WO97/05900, for example, exosomes may be isolated from biological samples by ultracentrifugation and their content, e.g., RNAs can be purified with the TRIZOL reagent (Invitrogen, Carlsbad, CA) according to the protocol provided by the manufacturer and identified after reverse transcription to cDNA by PCR using specific mRNA primers as described in the subsections "Exosome isolation" and "RNA Extraction and Reverse Transription" on pages 4 to 5 of Gallo et al. (2012), PLoS One.;7(3):e30679, the disclosure content of which is hereby incorporated by reference. RNAs can be isolated by Exosome RNA Isolation Kits offered by several manufacturers, such as Norgen Biotek Corp (Thorold, CANADA), e.g., Norgen's Urine Exosome RNA Isolation Kit (Norgen; Cat. No. 47200) for isolation and enrichment of exosomes from urine and tissue culture media, or Plasma/Serum Exosome RNA Isolation Kit (Norgen; Cat. No. 49200) for isolation and enrichment of exosomal RNA from of plasma, serum and ascitic fluid in accordance to the manual of the supplier. Furthermore, exosomes can be isolated instead of ultracentrifugation by the use of further kits, such as ExoQuick-TC^{™} from Gentaur (Kampenhout, Belgium) in accordance to the manual of the supplier. Methods for producing artificial exosomes are known in the art as well. Such artificial exosomes can be derived from coated liposomes as described in De La Peña et al., J. Immunol. Methods. 344 (2009), 121-132.

Lipid nanoparticles are spherical vesicles made of ionizable lipids, which are positively charged at low pH (enabling RNA complexation) and neutral at physiological pH (reducing potential toxic effects, as compared with positively charged lipids, such as liposomes). Owing to their size and properties, lipid nanoparticles are taken up by cells via endocytosis, and the ionizability of the lipids at low pH (likely) enables endosomal escape, which allows release of the cargo into the cytoplasm; see, e.g., Garcia-Pinel et al., Nanomaterials (Basel). 2019 Apr; 9(4): 638.

In view of the intended uses of therapy of the RNAs/mRNAs and nanovesicles as defined herein, the present invention further also relates to a pharmaceutical composition comprising (as active ingredient) at least one mRNA molecule, or at least one nanovesicle as defined herein above and a pharmaceutically acceptable carrier.

In addition to RNAs/mRNAs and nanovesicles for the uses in vivo, as described hereinabove, the present invention further relates to an use of a RNA/mRNA molecule or a nanovesicle of as defined herein for regeneration and/or rejuvenation of cells in cell culture. By such uses, e.g., cells or tissues can be prepared or modified in vitro for a potential further use in a subject, e.g., after an implantation. Preferably, the present application relates to an use, wherein the cell culture comprises cells selected from the group consisting of bone marrow stem cells including hematopoietic stem cells and mesenchymal stem cells, cartilage, cartilage stem cells, smooth muscle cells, smooth muscle stem cells, hairy cells and stem cells of the inner ear, soft tissue stem cells and endometrial cells.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example, the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### EXAMPLES

### Example 1:

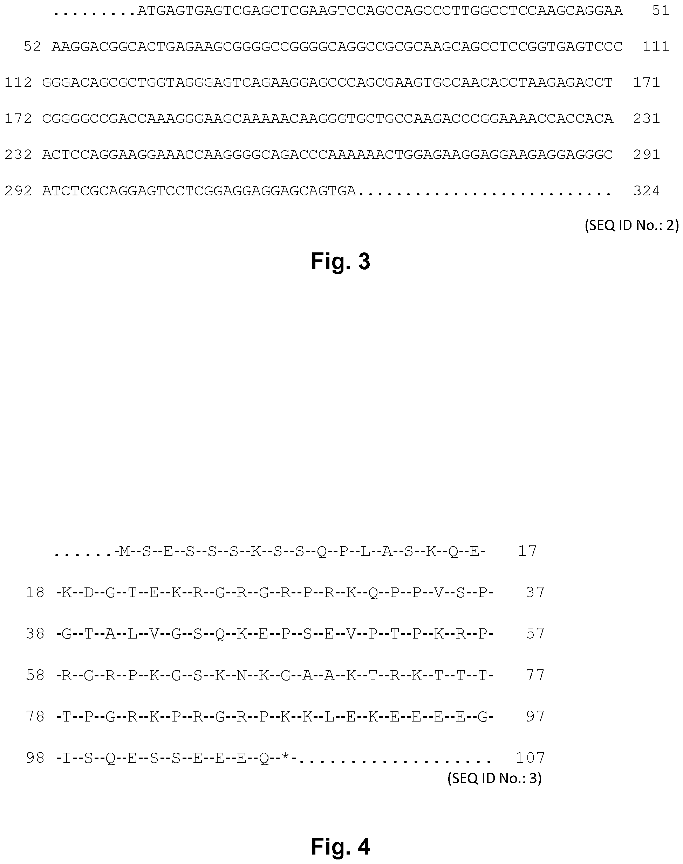

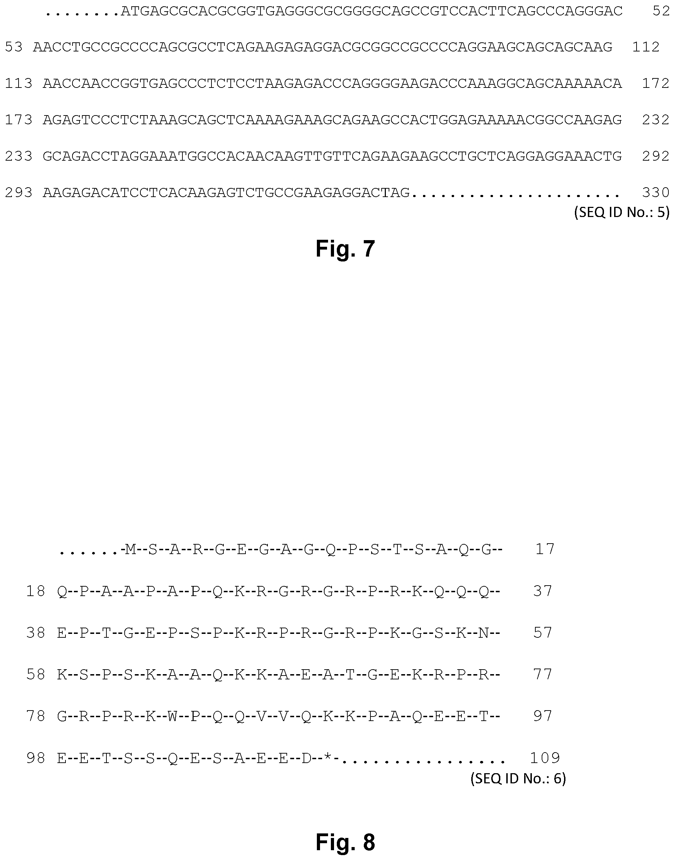

cDNA sequences and coding sequences of human *HMGA1* and *HMGA2* as well as their amino acid sequences have been depicted from the database and are shown in Figs. 1-8.

### Example 2: Preparation of exosomes and their HMGA2 mRNA-loading

Different techniques have been described allowing to load exosomes (Tsai et al., 2021) as well as artificial small extracellular vesicles as *e.g.* lipid nanoparticles (LNPs)(Zhang et al., 2021) with mRNA. Here, the inventors followed the protocol by Tsai et al. (2021) to prepare exosomes and load them with mRNA. Commercially available LNPs (DOTAP/DOPE Cationic Liposomes, lipid composition: DOTAP/DOPE (50:50 mol/mol) with a mean particle size of 80-100 nm and lipid concentration: 5 mM (3.61 mg/mL) (https://tribioscience.com/product/life-sciences/liposomes/cationic-liposomes-dotap-dopef50102/)) were also loaded following the same protocol. Briefly, lipid-coated mRNA of HMGA2 was used to load it into purified exosomes or into LNPs by mixing and incubation. So obtained exosomes or LNPs were used in the subsequent experiments then to deliver the mRNA to the target cells or tissues.

### Example 3: Induction of nuclear overexpression of HMGA2 after transfer of HMGA2 mRNA-constructs to explants of porcine deep fascia and cartilageneous tissue using loaded exosomes

*HMGA2* mRNA-constructs were loaded to exosomes and to lipid nanoparticles (LNPs) according to routine procedures as described above. Of note, protocols to load the vesicles by electroporation have also been described in the literature.

Specimens of porcine cartilage and deep fascia were taken under sterile conditions, minced into small explants with a diameter of about one millimeter, and incubated overnight in Roswell Park Memorial Institute (RPMI) 1640 medium. Then, the formulations of vesicles (exosomes, LNPs) loaded with full-lenth mRNA as well as mRNA lacking part of the 3 UTR between nucleotides 1101-1669 (see example 4) were added to these in vitro explants and incubated for another 12 hours allowing mRNA uptake as well as protein expression. Formulations of vesicles with mRNA containing only the coding part of exons 4 to 5 of *HMGA2* served as negative controls.

Immunohistochemical analysis of tissue sections (porcine cartilage and skin) mounted on slides was performed as described elsewhere (Kloth et al., 2015). Briefly, slides utilized for the immunohistochemical analysis were produced using histological sections (4 micrometer). Immunohistochemical staining for HMGA2 (rabbit polyclonal anti-HMGA2-P3, Biocheck, Inc., Forster City, USA) was performed using a detection kit (DAKO ChemMate; DAKO, Glostrup, Denmark) and a semiautomated stainer (DAKO; TechMate) according to the specifications of the manufacturer. For antigen retrieval, the slides were treated in a PT Link module (DAKO) using the EnVision^{™} FLEX Target Retrieval Solution, low pH (DAKO). The antibody dilution used was 1:1000. As to the staining sections of human term placenta were used as a positive control (strong expression of HMGA2) whereas the negative control for staining was performed by omission of the primary antibody.

Interpretation of HMGA2 staining was done using a Zeiss Axioplan (Carl Zeiss Microscopy GmbH, Göttingen, Germany) microscope. Immunoreactivity in the nucleus was considered positive while perinuclear granulation in cytoplasm that was observed occasionally was not considered for scoring. On each slide, three high-power fields were rated. The extent of staining was scored by multiplying intensity of staining (0: no staining, 0.5: very weak, 1: weak, 1.5: weak-moderate, 2: moderate, 2.5: moderate-strong and 3: strong) by percentage of stained nuclei cells. As a negative control, explants were incubated under the same conditions with vesicles that had not been loaded with mRNA.

Overall, both types of vesicles were able to induce clear, *i.e.,* moderate nuclear HMGA2 expression compared to the negative controls that did not reveal nuclear staining. Accordingly, both types of vesicles have been shown as applicable for the delivery of HMGA2 encoding molecules to the target cells and tissues and for the induction of expression of the encoded protein therein.

### Example 4: Let-7 binding sites

Let-7 binding sites can be detected by appropriate programmes. Herein, mirDB has been used (http://www.mirdb.org/mirdb/index.html).

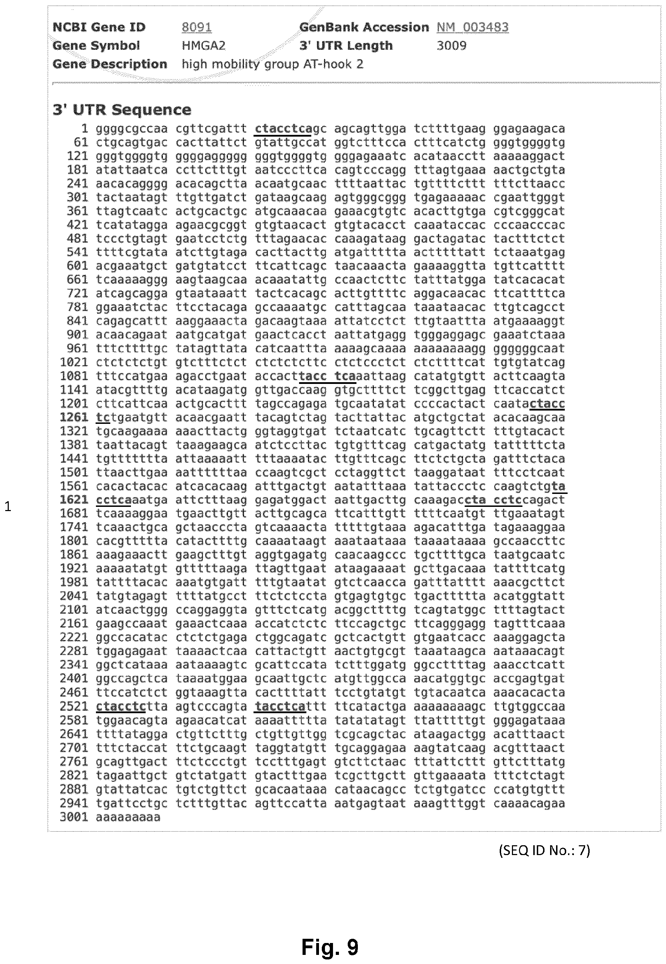

According to this analysis, the 3'UTR of *HMGA2* is showing several high affinity binding sites for miRNAs of the let-7 family *i.e.* let-7f-5p, let-7d-5p, let-7b-5p, let-7g-5p, let-7a-5p, let-7i-5p, let-7e-5p, let-7c-5p. The seed locations are depicted in the following figure (Fig. 9)(21, 1107, 1256, 1619, 1668, 2521. 2541) and apply to the miRNAs as mentioned above. Modification of these sites can be used to lower the binding of the miRNAs of the let-7 family to the 3'UTR of *HMGA2* and subsequently for induction of HMGA2 expression.

### Example 5: Modification of mRNA by alteration of let-7 target sites.

Removal and mutation of let7 binding sites can be performed as described previously (*e.g.* Mayr et al., 2007). Briefly, UTR with point mutations disrupting all seven target sites described above (example 4) or single of these target sizes have been synthesized. To disrupt the target sites nucleotides that paired to nucleotides 3 and 5 of let-7 were substituted. These UTR fragments were then cloned back into a vector containing the HMGA2 cDNA resulting in a total of eight different constructs (with seven individually disrupted target sites and one with all target sites that had been disrupted). Overall, disruption of all seven target sites lead to a significantly enhanced expression of HMGA2 in the nuclei of the explants taken from fascia as well as from cartilageous tissue as revealed by the score for nuclear positivity.

### References

Bai J, Yokomizo-Nakano T, Kubota S, Sun Y, Kanai A, Iimori M, Harada H, Iwama A, Sashida G. Overexpression of Hmga2 activates Igf2bp2 and remodels transcriptional program of Tet2-deficient stem cells in myeloid transformation. Oncogene. 2021 Feb;40(8):1531-1541. doi: 10.1038/s41388-020-01629-w. Epub 2021 Jan 15. PMID: 33452460.
Bansal S, Perincheri S, Fleming T, Poulson C, Tiffany B, Bremner RM, Mohanakumar T. Cutting Edge: Circulating Exosomes with COVID Spike Protein Are Induced by BNT162b2 (Pfizer-BioNTech) Vaccination prior to Development of Antibodies: A Novel Mechanism for Immune Activation by mRNA Vaccines. J Immunol. 2021 Nov 15;207(10):2405-2410. doi: 10.4049/jimmunol.2100637. Epub 2021 Oct 15. PMID: 34654691.
Cari L, Naghavi Alhosseini M, Mencacci A, Migliorati G, Nocentini G. Differences in the Expression Levels of SARS-CoV-2 Spike Protein in Cells Treated with mRNA-Based COVID-19 Vaccines: A Study on Vaccines from the Real World. Vaccines (Basel). 2023 Apr 21;11(4):879. doi: 10.3390/vaccines11040879. PMID: 37112792; PMCID: PMC10144021.
Dahlén A, Mertens F, Rydholm A, Brosjö O, Wejde J, Mandahl N, Panagopoulos I. Fusion, disruption, and expression of HMGA2 in bone and soft tissue chondromas. Mod Pathol. 2003 Nov;16(11):1132-40. doi: 10.1097/01.MP.0000092954.42656.94. PMID: 14614053.
Jouan Y, Bouchemla Z, Bardèche-Trystram B, Sana J, Andrique C, Ea HK, Richette P, Latourte A, Cohen-Solal M, Hay E. Lin28a induces SOX9 and chondrocyte reprogramming via HMGA2 and blunts cartilage loss in mice. Sci Adv. 2022 Aug 26;8(34):eabn3106. doi: 10.1126/sciadv.abn3106. Epub 2022 Aug 26. PMID: 36026443; PMCID: PMC9417174.
Kazmierczak B, Meyer-Bolte K, Tran KH, Wöckel W, Breightman I, Rosigkeit J, Bartnitzke S, Bullerdiek J. A high frequency of tumors with rearrangements of genes of the HMGI(Y) family in a series of 191 pulmonary chondroid hamartomas. Genes Chromosomes Cancer. 1999 Oct;26(2):125-33. PMID: 10469450.
Kloth L, Gottlieb A, Helmke B, Wosniok W, Löning T, Burchardt K, Belge G, Günther K, Bullerdiek J. HMGA2 expression distinguishes between different types of postpubertal testicular germ cell tumour. J Pathol Clin Res. 2015 Sep 12;1(4):239-51. doi: 10.1002/cjp2.26. PMID: 27499908; PMCID: PMC4939894.
Leonhardt C, Schwake G, Stögbauer TR, Rappl S, Kuhr JT, Ligon TS, Rädler JO. Single-cell mRNA transfection studies: delivery, kinetics and statistics by numbers. Nanomedicine. 2014 May;10(4):679-88. doi: 10.1016/j.nano.2013.11.008. Epub 2013 Dec 9. PMID: 24333584.
Mayr C, Hemann MT, Bartel DP. Disrupting the pairing between let-7 and Hmga2 enhances oncogenic transformation. Science. 2007 Mar 16;315(5818):1576-9. doi: 10.1126/science. 1137999. Epub 2007 Feb 22. PMID: 17322030; PMCID: PMC2556962.
Orlandini von Niessen AG, Poleganov MA, Rechner C, Plaschke A, Kranz LM, Fesser S, Diken M, Löwer M, Vallazza B, Beissert T, Bukur V, Kuhn AN, Türeci Ö, Sahin U. Improving mRNA-Based Therapeutic Gene Delivery by Expression-Augmenting 3' UTRs Identified by Cellular Library Screening. Mol Ther. 2019 Apr 10;27(4):824-836. doi: 10.1016/j.ymthe.2018.12.011. Epub 2018 Dec 18. PMID: 30638957; PMCID: PMC6453560.
Schoenmakers EF, Wanschura S, Mols R, Bullerdiek J, Van den Berghe H, Van de Ven WJ. Recurrent rearrangements in the high mobility group protein gene, HMGI-C, in benign mesenchymal tumours. Nat Genet. 1995 Aug;10(4):436-44. doi: 10.1038/ng0895-436. PMID: 7670494.
Smeti I, Watabe I, Savary E, Fontbonne A, Zine A. HMGA2, the architectural transcription factor high mobility group, is expressed in the developing and mature mouse cochlea. PLoS One. 2014 Feb 14;9(2):e88757. doi: 10.1371/journal.pone.0088757.
Tsai SJ, Atai NA, Cacciottolo M, Nice J, Salehi A, Guo C, Sedgwick A, Kanagavelu S, Gould SJ. Exosome-mediated mRNA delivery in vivo is safe and can be used to induce SARS-CoV-2 immunity. J Biol Chem. 2021 Nov;297(5):101266. doi: 10.1016/j.jbc.2021.101266. Epub 2021 Oct 1. PMID: 34600888; PMCID: PMC8483990.
Zhang Y, Sun C, Wang C, Jankovic KE, Dong Y. Lipids and Lipid Derivatives for RNA Delivery. Chem Rev. 2021 Oct 27;121(20):12181-12277. doi: 10.1021/acs.chemrev.1c00244. Epub 2021 Jul 19. PMID: 34279087; PMCID: PMC10088400.

## Claims

1. mRNA molecule encoding HMGA2 or part thereof containing at least exons 1-3 of the cDNA with the SEQ ID No.: 4 for use in cell regeneration.

2. mRNA molecule encoding HMGA1 or part thereof containing at least exons 1-3 of the cDNA with the SEQ ID NO.: 1 for use in cell regeneration.

3. mRNA molecule for use in cell regeneration according to claim 1 or 2, wherein the regenerated cells are selected from bone marrow stem cells and cartilage cells.

4. mRNA molecule according to claim 1 or 2 for use in cell rejuvenation.

5. mRNA molecule for use according to claim 1 and 2, wherein the regenerated cells are selected from the group consisting of bone marrow stem cells including hematopoietic stem cells and mesenchymal stem cells, cartilage cells, cartilage stem cells, smooth muscle cells, smooth muscle stem cells, hairy cells and stem cells of the inner ear, soft tissue stem cells, and the rejuvenated cells are endometrium cells.

6. mRNA molecule of anyone of the preceding claims for use in treatment of diseases related to cartilage loss.

7. mRNA molecule for use according to claim 6, wherein the diseases are selected from the group consisting of arthrosis, arthritis, rheumatoid arthritis, inflammatory arthropathies, joint injuries, osteoarthritis, systemic lupus erythematosus, seronegative spondyloarthropathies, herniation, achondroplasia, relapsing polychondritis, osteochondritis dissecans, relapsing polychondritis.

8. mRNA molecule for use according to anyone of claims 1-7, wherein at least 10 % uridine of the natural mRNA has been substituted by N1-methyl-pseudouridine.

9. mRNA molecule for use according to anyone of claims 1-7 where uridines of the natural mRNA have been substituted by residues with lower immunogenicity.

10. mRNA molecule for use according to anyone of claims 1-9, wherein at least one of the let-7 binding sites has been removed or mutated.

11. Nanovesicle (NV) containing at least one mRNA molecule or modified mRNA molecule according to anyone of claims 1 to 10.

12. Nanovesicle containing any of the mRNAs or modified mRNAs according to claims 1 to 5, wherein the nanovesicle is selected from the group consisting of exosomes, artificial exosomes, lipid-based nanoparticles, liposomes, niosomes, ethosomes, and cell derived nanovesicles.

13. Pharmaceutical composition comprising (as active ingredient) at least one mRNA molecule of the claims 1 to 10, or at least one nanovesicle of claim 11 or 12 and a pharmaceutically acceptable carrier.

14. Use of a mRNA molecule according to any one of claims 1 to 10 or a nanovesicle of claim 11 or 12 for regeneration or rejuvenation of cells in cell culture.

15. Use according to claim 14, wherein the cell culture comprises cells selected from the group consisting of bone marrow stem cells including hematopoietic stem cells and mesenchymal stem cells, cartilage, cartilage stem cells, smooth muscle cells, smooth muscle stem cells, hairy cells and stem cells of the inner ear, soft tissue stem cells and endometrial cells.
